(19) Europäisches Patentamt
European Patent Office
Office européen des brevets

(11) Publication number: 0 539 405 B1

# EUROPEAN PATENT SPECIFICATION

(45) Date of publication of patent specification: 28.06.95

(51) Int. Cl.⁶: **C07K 14/155**, A61K 39/21, G01N 33/569

(21) Application number: **91912338.0**

(22) Date of filing: **10.07.91**

(86) International application number: **PCT/CA91/00238**

(87) International publication number: **WO 92/01713 (06.02.92 92/04)**

(54) **PEPTIDES AND ANALOGUES AND MIXTURES THEREOF FOR DETECTING ANTIBODIES TO HTLV-I AND HTLV-II VIRUSES.**

(30) Priority: **18.07.90 US 554258**
**20.03.91 US 672483**

(43) Date of publication of application:
**05.05.93 Bulletin 93/18**

(45) Publication of the grant of the patent:
**28.06.95 Bulletin 95/26**

(84) Designated Contracting States:
**AT BE CH DE DK ES FR GB GR IT LI LU NL SE**

(56) References cited:
**EP-A- 0 107 053**
**WO-A-89/08664**
**WO-A-90/08162**
**WO-A-90/10231**

(73) Proprietor: **BIOCHEM IMMUNOSYSTEMS INC.**
**10900 Hamon Street**
**Montreal**
**Ouebec H3M 3A2 (CA)**

(72) Inventor: **LACROIX, Martial**
**9025 Richmond**
**Brossard, Ouebec J4X 2R9 (CA)**
Inventor: **ZREIN, Maan**
**224 Boulevard des Prairies**
**Laval, Ouebec H7N 2T9 (CA)**

(74) Representative: **Ritter, Stephen David et al**
**Mathys & Squire**
**100 Grays Inn Road**
**London WC1X 8AL (GB)**

Journal of Immunology, vol. 137, no. 9, 1 November 1986, The American Association of Immunologists, Baltimore, US), T D Copeland et al.:"Envelope proteins of human T cell leukemia virus type I : Characterisation by antisera to synthetic peptides and identification of a natural epitope", pages 2945-2951.

Journal of Immunology, vol. 142, no. 3, 1 February 1989, The American Association of Immunologists, (Baltimore, US), T J Palker et al.:"Mapping of immunogenic regions of human T cell leukemia virus type I (HTLV-I) gp46 and gp21 envelope glycoproteins with env-encoded synthetic peptides and a monoclonal antibody to gp46", pages 971-978

**Description**

This application is a continuation-in-part of United States patent application Serial No. 07/554,258 filed July 18, 1990.

TECHNICAL FIELD OF THE INVENTION

This invention relates to novel linear peptides and mixtures and chemical combinations thereof useful for detecting and quantifying HTLV-I and HTLV-II infections. These peptides are also useful in vaccines against HTLV-I and HTLV-II viral infections.

BACKGROUND OF THE INVENTION

Human T-cell leukemia virus subgroup I (HTLV-I) is a retrovirus closely associated with adult T-cell leukemia/lymphoma (ATL) (Poiesz et al., **1980**, Proc. Natl. Acad. Sci. U.S.A., 77, 7415-7419). It is also linked with neurological diseases designated as tropical spastic paraparesis and HTLV-I-associated myelopathy (HAM) (Gessain et al., **1985**, Lancet ii, 407-410). Human T-cell leukemia virus subgroup II (HTLV-II) was first isolated from a patient with T-cell hairy cell leukemia (Kalyanaraman et al., **1982**, Science, 218, 571-573). It is associated with a T-cell variant of hairy cell leukemia.

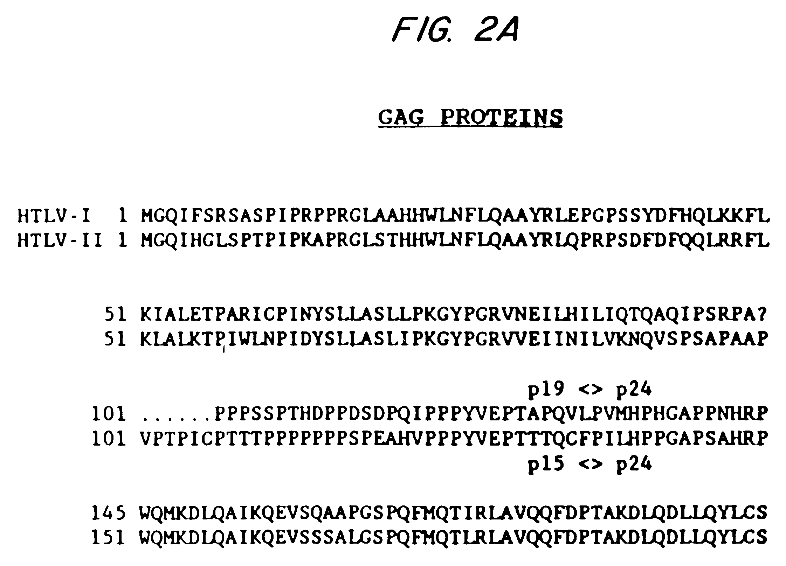

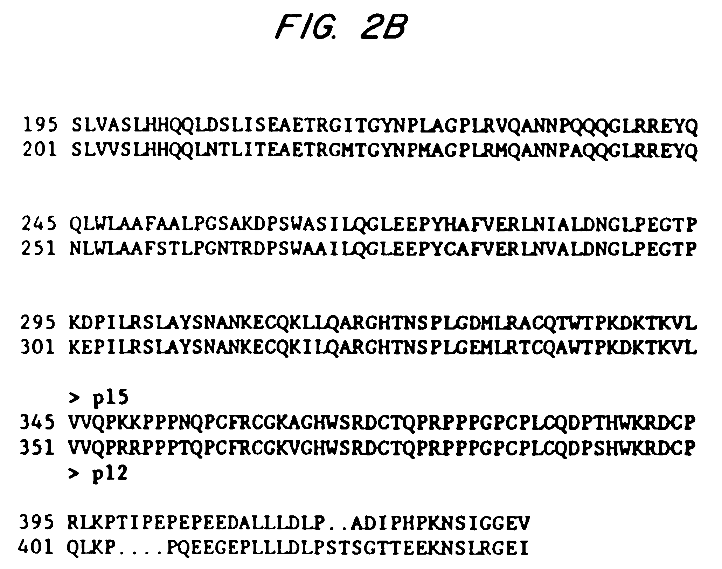

The HTLV-I and HTLV-II genomes exist as proviruses in the human chromosomal DNA of leukemia cells. Their complete nucleotide sequences have been elucidated (Seiki et al., **1983**, Proc. Natl. Acad. Sci. U.S.A., 80, 3618-3622; Shimotohno et al., **1985**, Ibid., 82, 3101-3105). Like other retroviruses, the HTLV-I and HTLV-II genomes are flanked by LTR (long-terminal-repeats) structures which are believed to play an essential role in the integration of the proviral DNA into the host chromosomal DNA. Four major genes have been identified and occupy the following relative positions in the genome: LTR-**gag-pol-env-pX**-LTR (Seiki et al.; Shaw et al., **1984**, Proc. Natl. Acad. Sci., 81, 4544-4548). The **gag** gene codes for a 48,000-dalton-precursor protein (often referred to as p53) consisting of 429 amino acids (433 for HTLV-II). This precursor protein is cleaved into at least three smaller proteins (Figure 2). The **pol** gene codes for the viral reverse transcriptase. The **env** gene presumably codes for a 54,000-dalton protein which is glycosylated and later cleaved into two glycoproteins named gp46 and gp21. (Slightly different molecular weights have been reported for what are most probably the same **env** gene protein products). The last coding region is called **pX** and is believed to code for four proteins ($tax_1$, $tax_2$, **rex$_1$**, **rex$_2$**) involved in gene expression and regulation.

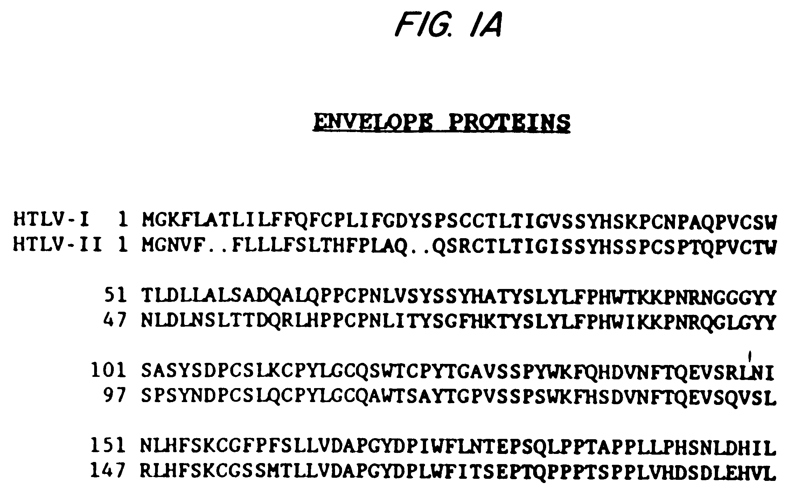

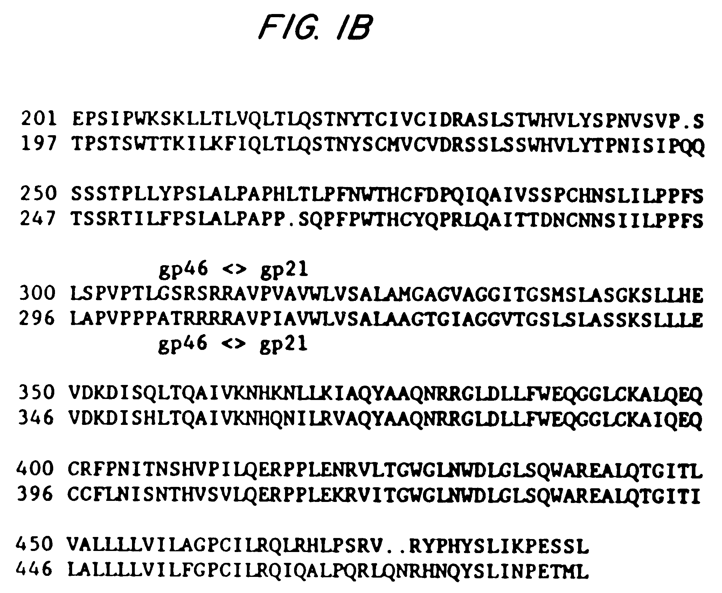

As depicted in Figures 1 and 2, the **env** proteins and the **gag** proteins of HTLV-I and of HTLV-II share a high degree of amino acid sequence homology. Furthermore, serologic cross-reactivity has been reported between the proteins of HTLV-I and those of HTLV-II (Lee et al., **1984**, Proc. Natl. Acad. Sci., 81, 7579-7583). Despite this structural resemblance and cross-reactivity, some regions of these **env** and **gag** proteins are recognized only by antibodies present in a patient infected by either the HTLV-I or the HTLV-II virus.

HTLV-I and HTLV-II both differ from the human immunodeficiency viruses (HIVs) in their morphologic and genetic structures. As a result, antibodies to the HIV proteins should not cross-react with HTLV-I and HTLV-II antigens. However, some serum samples taken from patients with AIDS have shown some degree of cross-reactivity with HTLV antigens (Essex et al., **1983**, Science, 220, 859-862).

Adult T-cell leukemia/lymphoma (ATL) occurs mainly in Southwestern Japan, the Caribbean basin and parts of Central and South America. In those regions, seroprevalence varies between 5% and 15% in the general population and reaches 30% in older age groups. In the United States, HTLV-I/II infections are mainly present in intravenous drug users (IVDU). In a recent American Red Cross survey (Williams et al., **1988**, Science, 240, 643-646), antibodies to HTLV-I could be detected in 10 of 39,898 random blood donors in eight U.S. cities; this represents a seroprevalence rate of 0.025%. A similar study involving 3158 individuals from Northern Egypt led to the identification of two carriers (prevalence rate of 0.06%) (El-Farrash et al., **1988**, Microbiol. Immunol., 32, 981-984). In these two studies, distinction between infection with HTLV-I and HTLV-II was not clearly established. These data indicate that there is a need for a reliable test to screen all blood samples destined for blood banks in order to avoid the inadvertent spread of the virus to blood product recipients.

There have been several attempts in the prior art to develop such tests. None has been successful in detecting all serum and plasma samples that are part of a well characterized commercial panel of HTLV infected fluids. Furthermore, none is able to detect HTLV infection at very low levels, thereby, ensuring safety of the blood supply and prompt and early treatment of HTLV infections.

Saxinger et al. (**1984**, Science, 225, 1473-1476) has reported the use of the HTLV-I particle as the immunoadsorbent in an enzyme immunoassay (EIA) for the detection of antibodies to the virus. In an improvement to this first generation test, specific HTLV antigens have been used instead of viral particles as the immunoadsorbent. For example, Samuel et al. (**1984**, Science, 225, 1094-1097) refers to antigens obtained by recombinant DNA technology. These detected each of 11 sera shown to contain antibodies to HTLV-I by a whole viral lysate-based EIA similar to the one developed by Saxinger et al. Slamon et al. (PCT/US85/01803) refer to assays using polypeptides and fragments thereof associated with immunogenic sites present on proteins of the **pX** region of HTLV-I and HTLV-II. The reported accuracy of these assays ranged between 77% and 87%. Fukui et al. (European Patent Application 87116787) refers to assays using polypeptides encoded by a fused gene comprising all or a part of the gag gene and all or a part of the env gene. They reported a sensitivity of 100% (57/57) with no false-positives.

Although these results look impressive, they are likely not repeatable with sera, like those most usual in the United States, which are characterized by much lower antibody titers than the Japanese sera used to validate the above assays.

Assays which use peptides derived from HTLV-I or HTLV-II proteins have also been reported. For example, Palker et al. (**1989**, J. Immunol., 142, 971-978) reports that antibodies in sera from 28 out of 36 patients (78%) reacted with a peptide spanning amino acids 190 to 209 of the HTLV-I envelope (peptide 4a; **env** 190-209).*Ten of 35 sera samples (29%) reacted with peptide 6 (**env** 296-312) and 6 out of 33 (18%) bound to peptide 7 (**env** 374-392). Palker et al. (**1986**, J. Immunol., 136, 2393-2397) also refers to a peptide (SP-71; **gag** 120-130) that reacted with 16 out of 18 HTLV-I seropositive samples.

A peptide (SP-70; **env** 296-306) has been reported by Copeland et al. (**1986**, J. Immunol., 137, 2945-2951) to recognize antibodies from 4 out of 12 individuals seropositive for HTLV-I.

Wang et al. (US Patent 4,833,071) refers to three overlapping linear peptides spanning regions of the transmembrane protein (gp21) of HTLV-I. These peptides (**env** 381-400, **env** 377-400 and **env** 378-393), when used in a mixture, detected 102 out of 102 serum samples from patients with ATL and 5 out of 30 patients with AIDS/ARC. No immunoreactivity was found against sera from 12 normal subjects or from 12 patients with autoimmune diseases.

Reyes (PCT/W089/06543) refers to a non-glycosylated, 41-amino acid recombinant peptide antigen derived from the gp46 of HTLV-I. This recombinant peptide, **env**(163-203), is reported to have been used in a solid-phase assay for the determination of serum antibodies in six patients with HTLV-I infection.

Vahlne (PCT/W089/08664) refers to four synthetic peptides, A, B, C and H, spanning **env**(381-404), **env**(273-293), **env**(223-242) and **env**(176-199) of the envelope protein of HTLV-I. From the examples provided, it appears that only peptide A was useful in an ELISA for the detection of specific antibodies to HTLV-I present in the samples tested.

None of the above-described assays has demonstrated the high specificity (no false positives) and high sensitivity (detection of all positives, even when the sera contains very low levels of HTLV antibodies) necessary to ensure that HTLV infected blood products do not enter blood banks and that infected individuals seek prompt and early treatment. The peptides of this invention remedy these failures.

SUMMARY OF THE INVENTION

The peptides of this invention are selected from the group consisting of:
(i) peptides having the formula:

a - X1 - b

wherein:
X1 is a sequence of at least six amino acids taken as a block from $AA_{283}$-$AA_{309}$ of the gp46 **env** protein (HTLV-I) and analogues thereof;
a is an amino terminus, one to eight amino acids or a substituent effective to facilitate coupling or to improve the immunogenic or antigenic activity of the peptide; and
b is a carboxy terminus, one to eight amino acids or a substituent effective to facilitate coupling or to improve the immunogenic or antigenic activity of the peptide;
(ii) peptides having the formula:

* In this application, the amino acid sequence and numbering published by Seiki et al. (supra.) and by Shimotohno et al. (supra.) for the HTLV-I and HTLV-II gene products are used (for ease of reference only).

a - X2 - b

wherein:

X2 is a sequence of at least six amino acids taken as a block from $AA_{273}$-$AA_{305}$ of the gp46 **env** protein (HTLV-II), and analogues thereof and a and b are as defined above;

(iii) peptides having the formula:

a - Z1 - b

wherein:

Z1 is a sequence of at least six amino acids taken as a block from $AA_{236}$-$AA_{257}$ of the **gag** p24 protein (HTLV-I), and analogues thereof and a and b are as defined above;

(iv) peptides having the formula:

a - Z2 - b

wherein:

Z2 is a sequence of at least six amino acids taken as a block from $AA_{242}$-$AA_{263}$ of the **gag** p24 protein (HTLV-II), and analogues thereof and a and b are as defined above;

(v) peptides having the formula:

a - B2 - b

wherein:

B2 is a sequence of at least six amino acids taken as a block from $AA_{171}$-$AA_{207}$ of the gp46 **env** protein (HTLV-II), and analogues thereof and a and b are as defined above; and

(vi) tandem peptides having the formula:

$$a-((((J)_n\text{-}c)_o - ((U)_p)_q - d)_r)_s - b$$

wherein:

J and U are sequences of at least six amino acids taken as a block from sequences independently selected from the group consisting of $AA_{193}$-$AA_{210}$ or $AA_{283}$-$AA_{309}$ of the gp46 **env** protein (HTLV-I); $AA_{236}$-$AA_{257}$ of the p24 **gag** protein (HTLV-I); $AA_{120}$-$AA_{130}$ of the p19 **gag** protein (HTLV-I); $AA_{171}$-$AA_{207}$ or $AA_{273}$-$AA_{305}$ of the gp46 **env** protein (HTLV-II); $AA_{242}$-$AA_{263}$ of the p24 **gag** protein (HTLV-II) and $AA_{126}$-$AA_{136}$ of the p19 **gag** protein (HTLV-II); and analogues thereof;

a and b are as defined above;

c and d, if present, are independently selected from one to eight amino acids or a substituent effective to facilitate coupling or to improve the immunogenic or antigenic activity of the peptide;

n = 1-5;

o = 1-5 if c is present, otherwise o = 1;

p = 1-5;

q = 1-5;

r = 1-5 if d is present, otherwise r = 1; and

s = 1-5.

The peptides and mixtures thereof of this invention are useful for the screening of blood and body fluids for the presence of HTLV-I and HTLV-II infraction and in the preparation of safe, effective vaccines against HTLV-I and HTLV-II infections. For example, the peptides of this invention and mixtures thereof are useful in a wide variety of specific binding assays for the detection of antibodies to HTLV-I and HTLV-II, as immunogens for eliciting antibodies useful for the detection of HTLV-I and HTLV-II antigens and in the preparation of vaccines against HTLV-I and HTLV-II viral infections.

BRIEF DESCRIPTION OF THE DRAWINGS

Figure 1 depicts the amino acid sequence of the **env** gene products (gp46 and gp21) of HTLV-I and HTLV-II. The amino acid residues of the sequence are given using the following single letter code: A = ala, C = cys, D = asp, E = glu, F = phe, G = gly, H = his, I = ile, K = lys, L = leu, M = met, N = asn, P = pro, Q = gln, R = arg, S = ser, T = thr, V = val, W = trp, Y = tyr. The "..." are spaces added to align the depicted sequences as closely as possible.

Figure 2 depicts the amino acid sequence of the **gag** gene products of HTLV-I and HTLV-II. Again, the "..." are spaces added to align the depicted sequences as closely as possible.

DESCRIPTION OF THE INVENTION

The present invention provides novel peptides and analogues thereof corresponding to immunodominant regions of the **env** gene and **gag** gene products of HTLV-I and HTLV-II. It also provides mixtures and chemical combinations of those peptides and analogues. As will be plain from the following description, these peptides, analogues, mixtures and chemical combinations are useful in a wide variety of diagnostic and preventive methods, means and compositions with respect to HTLV-I and HTLV-II and the infections caused by them. Some of the peptides, analogues, mixtures and chemical combinations of this invention are also capable of distinguishing with which virus, HTLV-I or HTLV-II, a particular patient is infected.

As set forth above, the peptides of this invention are selected from the group consisting of:

(i) peptides having the formula:

a - X1 - b

wherein:

X1 is a sequence of at least six amino acids taken as a block from $AA_{283}$-$AA_{309}$ of the gp46 **env** protein (HTLV-I), and analogues thereof;

a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide;

(ii) peptides having the formula:

a - X2 - b

wherein:

X2 is a sequence of at least six amino acids taken as a block from $AA_{273}$-$AA_{305}$ of the gp46 **env** protein (HTLV-II), and analogues thereof and a and b are as defined above;

(iii) peptides having the formula:

a - Z1 - b

wherein:

Z1 is a sequence of at least six amino acids taken as a block from $AA_{236}$-$AA_{257}$ of the **gag** p24 protein (HTLV-I), and analogues thereof and a and b are as defined above;

(iv) peptides having the formula:

a - Z2 - b

wherein:

Z2 is a sequence of at least six amino acids taken as a block from $AA_{242}$-$AA_{263}$ of the **gag** p24 protein (HTLV-II), and analogues thereof and a and b are as defined above;

(v) peptides having the formula:

a - B2 - b

wherein:

B2 is a sequence of at least six amino acids taken as a block from $AA_{171}$-$AA_{207}$ of the gp46 **env** protein (HTLV-II), and analogues thereof and a and b are as defined above; and

(vi) tandem peptides having the formula:

a-$((((J)_n$-c$)_o$ - $((U)_p)_q$ - d$)_r)_s$ - b

wherein:

J and U are sequences of at least six amino acids taken as a block from sequences independently selected from the group consisting of $AA_{193}$-$AA_{210}$ or $AA_{283}$-$AA_{309}$ of the gp46 **env** protein (HTLV-I); $AA_{236}$-$AA_{257}$ of the p24 **gag** protein (HTLV-I); $AA_{120}$-$AA_{130}$ of the p19 gag protein (HTLV-I); $AA_{171}$-$AA_{207}$ or $AA_{273}$-$AA_{305}$ of the gp46 **env** protein (HTLV-II); $AA_{242}$-$AA_{263}$ of the p24 **gag** protein (HTLV-II) and $AA_{126}$-$AA_{130}$ of the p19 **gag** protein (HTLV-II); and analogues thereof;

a and b are as defined above;

c and d, if present, are independently selected from one to eight amino acids or a substituent effective to facilitate coupling or to improve the immunogenic or antigenic activity of the peptide;

n = 1-5;

o = 1-5 if c is present, otherwise o = 1;

p = 1-5;

q = 1-5;

r = 1-5 if d is present, otherwise r = 1; and

s = 1-5.

As used herein "analogues" denote amino acid insertions, deletions, substitutions and modifications at one or more sites in the peptide chain in that portion of it that consists of the block of the naturally occurring HTLV-I or HTLV-II amino acid sequences.

Preferred modifications and substitutions to the native amino acid sequence of the peptides of this invention are conservative ones (i.e., those having minimal influence on the secondary structure and hydropathic nature of the peptide). These include substitutions such as those described by Dayhoff in the Atlas of Protein Sequence and Structure 5, **1978** and by Argos in EMBO J. 8, 779-785, **1989**. For example, amino acids belonging to one of the following groups represent conservative changes: ala, pro, gly, glu, asp, gln, asn, ser, thr; cys, ser, tyr, thr; val, ile, leu, met, ala, phe; lys, arg, his; and phe, tyr, trp, his. In like manner, methionine, an amino acid which is prone to oxidation may be replaced by norleucine. The preferred substitutions also include substitutions of D-isomers for the corresponding L-amino acids.

However, as described above, irrespective of such insertions, deletions, substitutions and modifications, the peptides of this invention must contain at least six amino acids taken as a block from one of the following: $AA_{283}$-$AA_{309}$ of the gp46 **env** protein (HTLV-I); $AA_{273}$-$AA_{305}$ of the gp46 product of the **env** gene (HTLV-II); $AA_{236}$-$AA_{257}$ of the p24 product of the **gag** protein (HTLV-I); $AA_{242}$-$AA_{263}$ of the p24 product of the **gag** protein (HTLV-II); and $AA_{171}$-$AA_{207}$ of the gp46 product of the **env** protein (HTLV-II). Likewise, the tandem peptides of this invention must contain at least six amino acids taken as a block from J and U as defined above.

Preferably, in the peptide formulas of this invention, block sequences X1, X2, Z1, Z2, B2, J and U are sequences of at least 10 amino acids. More preferably, they are sequences of at least 15 amino acids. Even more preferably, they are sequences of at least 20 amino acids and most preferably, they are sequences of at least 25 amino acids. Preferably, in the tandem peptide formula of this invention, n, o, p, q, r and s = 1-3. More preferably, n, o, p, q, r and s = 1-2.

The term "amino acid" as employed in this description (e.g., in the definition of a and b) except when referring to the amino acid sequence of the gene products of the HTLV-I or HTLV-II, encompasses all of the natural amino acids, those amino acids in their D-configurations, and the known non-native, synthetic, and modified amino acids, such as homocysteine, ornithine, norleucine and $\beta$-valine.

The term "block" as used herein refers to a sequence of amino acids identical to that present in a native polypeptide of HTLV-I or HTLV-II.

As reflected in (vi) above, it is within the scope of this invention to employ tandem peptides. These peptides may be homopolymers, copolymers or multimers. We have unexpectedly found that these tandem peptides show a very high degree of reactivity to HTLV antibodies. Physical mixtures of the peptides and tandem peptides of this invention are also within its scope.

Illustrative of peptides of this invention that are derived from gp46 of the env gene of HTLV-I is:

BCH-416 a-AIVSSPSHNSLILPPFSLSPVPTLGSR-b **env**(283-309)

wherein the cysteine residue in position 289 was replaced by a serine and also wherein a and b are as defined above.

Illustrative of peptides of this invention derived from gp46 of the **env** gene of HTLV-II are:

BCH-219 a-LVHDSDLEHVLTPSTSWTTKIL-b **env**(186-207)

BCH-404 a-TTDNSNNSIILPPFSLAPV-b **env**(281-299)

BCH-407 a-YQPRLQAITTDNSNNSIILPPFSLAPV-b **env**(273-299)

BCH-418 a-TTDNSNNSIILPPFSLAPVPPPATR-b **env**(281-305)

BCH-447 a-FITSEPTQPPPTSPPLVHDSDLEHVLTPSTS-b **env**(171-201)

BCH-486 a-TSEPTQPPPTSPPLVHDSDLEHVL-b **env**(173-196)

and
BCN-456 a-TSEPTQPPPTSPPLLVHDSDLEHVL-b **env**(173-186-L-187-196)
wherein a and b are as defined above.

Illustrative of the peptides of this invention derived from p24 of the **gag** gene of HTLV-I is:
BCH-411 a-QQGLRREYQQLWLAAFAALPGS-b **gag**(236-257)
wherein a and b are as defined above.

Illustrative of the peptides derived from p24 of the **gag** gene of HTLV-II is:
BCH-412 a-QQGLRREYQNLWLAAFSTLPGN-b **gag**(242-263)
wherein a and b are as defined above.

The preferred tandem peptides of this invention are defined by the formula:

a - J - c - U - b

wherein a, b, c, J and U are as defined above. Most preferably, one of J and U is selected from an HTLV-I peptide and the other is selected from an HTLV-II peptide. Illustrative of these preferred tandem peptides is:
BCH-234 a-PHSNLDHILEPSIPWKSKPYVEPTAPQVL-b
wherein:

J is BCH-152 (HSNLDHILEPSIPWKSKL) (derived from the **env** gene of HTLV-I: **env** (193-210)) to which a Pro residue was added at the N-terminus (a) and a Leu residue at the C terminus was removed, c not being present and U being BCH-228 (PYVEPTAPQVL) (derived from the **gag** gene of HTLV-I: **gag** (120-130), the last 11 amino acids of p19 (Figure 2)).

The preferred peptides of this invention are BCH-219, BCH-407, BCH-416, BCH-418, BCH-447, BCH-456, BCH-486 and tandem peptide BCH-234. The most preferred peptides of this invention are BCH-416, BCH-456, BCH-486 and BCH-234. The preferred peptide mixture of this invention contains at least BCH-416. More preferably, it contains at least BCH-416 and BCH-234 and most preferably, it contains at least BCH-416, BCH-234 and BCH 456.

In addition to their use in HTLV-I and II diagnostic means, methods and compositions, the peptides of this invention may also be used to distinguish and HTLV-I infection from an HTLV-II infection. Such diagnostic distinction is done, for example, by testing the sample separately with an HTLV-I derived peptide of this invention, or mixture thereof with other HTLV-I peptides and with an HTLV-II derived peptide of this invention or mixture thereof with other HTLV-II peptides and determining which peptide reacts most strongly. The preferred peptides for such methods are BCH-234 for the identification of an HTLV-I infection and BCH-456 and BCH-486 for the identification of an HTLV-II infection.

The peptides of this invention and mixtures thereof are also useful in vaccines against HTLV infections. Antibody titers against HTLV-I and II are notoriously low indicating that these viruses are not very immunogenic or bear immunosuppressive epitopes dampening the normal immune response. Use of synthetic peptides mimicking only those epitopes responsible for the induction of neutralizing antibodies may, thus, be of value in the development of candidate synthetic vaccines. Therefore, it is also within the scope of this invention to use these peptides in vaccines against HTLV-I and HTLV-II infections.

An unexpected advantage of the peptides and mixtures of this invention is their high sensitivity. They are capable of detecting HTLV-I/II-specific antibodies in the majority, and sometimes in all, of confirmed seropositive samples present in a commercial panel sold for the assessment of the performance of anti-HTLV-I/II kits. Another advantage of the peptides and mixtures of this invention is their high level of specificity -- a minimal number of false positives. For example, using the above described peptide mixture (BCH-219, BCH-234 and BCH-416) no false-positive was recorded when 150 samples taken from the normal blood donor population were tested. Moreover, average ratio of O.D. recorded upon cut off was equal to 0.25 for these negative samples.

As set forth briefly above, it is often useful and certainly within the scope of this invention to modify the peptides of this invention in order to make the chosen peptide more useful as an immunodiagnostic reagent or as an active ingredient of a vaccine. Such changes, for example, include:

- addition of a cysteine residue to one or both terminals in order to facilitate coupling of the peptide to a suitable carrier with heterobifunctional cross-linking reagents such as sulfosuccinimidyl-4-(p-maleimidophenyl) butyrate, preferred reagents for effecting such linkages are sulfosuccinimidyl- sulfosuccinimidyl-4-(N-maleimidomethyl)cyclohexane-1-carboxylate and N-succinimidyl-3-(2-pyridyldithio) propionate;

- addition of 1 to 8 additional amino acids at one or both terminals of the peptide to facilitate linking of the peptides to each other, for coupling to a support or larger peptide or protein or for modifying the physical or chemical properties of the peptide. Examples of such changes are the addition of N- or C-terminal tyrosine, glutamic acid or aspartic acid as linkers via an esterification reaction and lysine which can be linked via Schiff base or amide formation. As described above such additional amino acids may include any of the natural amino acids, those amino acids in their D-configurations, and the known non-native, synthetic and modified amino acids; and

- derivatization of one or both terminals of the peptide by, for example, acylation or amidation. These modifications result in changes in the net charge on the peptide and can also facilitate covalent linking of the peptide to a solid support, a carrier or another peptide. Examples of the substituents effective to facilitate coupling or to improve the immunogenicity or antigenic activity of the peptide are $C_2$-$C_{16}$ acyl groups, polyethylene glycol and phospholipids.

To prepare the novel peptides of this invention any of the conventional peptide production methodologies may be used. These include synthesis, recombinant DNA technology and combinations thereof. We prefer solid phase synthesis. In that synthetic approach, the resin support may be any suitable resin conventionally employed in the art for the solid phase preparation of peptides. Preferably, it is a p-benzyloxyalcohol polystyrene or p-methylbenzydrylamine resin. Following the coupling of the first protected amino acid to the resin support, the amino protecting group is removed by standard methods conventionally employed in the art. After removal of the amino protecting group, the remaining protected amino acids and, if necessary, side chain protected amino acids are coupled, sequentially, in the desired order to obtain the chosen peptide. Alternatively, multiple amino acid groups may be coupled using solution methodology prior to coupling with the resin-supported amino acid sequence.

The selection of an appropriate coupling reagent follows established art. For instance, suitable coupling reagents are N,N'-diisopropylcarbodiimide or N,N'-dicyclohexylcarbodiimide (DCC) or benzotriazol-1-yloxy-tris(dimethylamino)phosphonium hexafluoro-phosphate either alone or preferably in the presence of 1-hydroxybenzotriazole. Another useful coupling procedure employs preformed symmetrical anhydrides of protected amino acids.

The necessary $\alpha$-amino protecting group employed for each amino acid introduced onto the growing polypeptide chain is preferably 9-fluorenylmethyloxycarbonyl (FMOC), although any other suitable protecting group may be employed as long as it does not degrade under the coupling conditions and is readily removable selectively in the presence of any other protecting group already present in the growing peptide chain.

The criteria for selecting protecting groups for the side chain amino acids are: (a) stability of the protecting group to the various reagents under reaction conditions selective for the removal of the $\alpha$-amino protecting group at each step of the synthesis; (b) retention of the protecting group's strategic properties (i.e., not be split off under coupling conditions) and (c) removability of protecting group easily upon conclusion of the peptide synthesis and under conditions that do not otherwise affect the peptide structure.

The fully protected resin-supported peptides are preferably cleaved from the p-benzyloxy alcohol resin with 50% to 60% solution of trifluoroacetic acid in methylene chloride for 1 to 6 hours at room temperature in the presence of appropriate scavengers such as anisole, thioanisole, ethyl methyl sulfide, 1,2-ethanedithiol and related reagents. Simultaneously, most acid labile side-chain protecting groups are removed. More acid resistant protecting groups are typically removed by HF treatment.

The peptides of the present invention are useful as diagnostic reagents for the detection and quantification of HTLV-I and HTLV-II-associated antibodies in accordance with methods well-known in the art. These include ELISA, hemagglutination, single-dot and multi-dot methods and assays.

A preferred convenient and classical technique for the determination of antibodies against the HTLV-I and HTLV-II using a peptide or a peptide mixture of this invention is an enzyme-linked immunosorbent assay (ELISA). In this assay, for example, a peptide or mixture of this invention is adsorbed onto, or covalently coupled to, the wells of a microtiter plate. The wells are then treated with the sera or analyte to be tested. After washing, anti-human IgG or anti-human IgM labeled with peroxidase is added to the wells. The determination of the peroxidase is performed with a corresponding substrate, e.g., 3,3',5,5'-tetra-methylbenzidine. Without departing from the usefulness of this illustrative assay, the peroxidase can be exchanged by another label, e.g., by a radioactive, fluorescence, chemiluminescence or infra-red emitting label.

Another method for the determination of antibodies against the HTLV-I and HTLV-II with the peptides and mixtures of this invention is an enzyme immunological test according to the so-called "Double-Antigen-Sandwich-Assay". This method is based on the work of Maiolini, as described in Immunological Methods, 20, 25-34, 1978. According to this method, the serum or other analyte to be tested is contacted with a solid phase on which a peptide of this invention has been coated (capture layer) and with a peptide of this invention which has been labeled with peroxidase or other label (probe layer).

The immunological reaction can be performed in one or two steps. If the immunological reaction is performed in two steps, then a washing step is typically carried out between the two incubations. After the immunological reaction or reactions, a washing step is also usually performed. Thereafter, the peroxidase or other signal is determined, e.g., using o-phenylene diamine for peroxidase. Other enzymes and chromogens, including those already described, can also be employed in this assay.

Suitable solid phases for use in the above-described assays and assay methods include organic and inorganic polymers, e.g., amylases, dextrans, natural or modified celluloses, polyethylene, polystyrene, polyacrylamides, agaroses, magnetite, porous glass powder, polyvinyldiene fluoride (kynar) and latex, the inner wall of test vessels (i.e., test tubes, titer plates or cuvettes of glass or artificial material) as well as the surface of solid bodies (i.e., rods of glass and artificial material, rods with terminal thickening, rods with terminal lobes or lamellae). Spheres of glass and artificial material are especially suitable as solid phase carriers.

The peptides and mixtures of this invention are not only useful in the determination and quantification of antibodies against the HTLV-I and HTLV-II. They are also useful for the determination and quantification of HTLV-I and HTLV-II antigens themselves because the peptides of this invention, either free, polymerized or conjugated to an appropriate carrier, are useful in eliciting antibodies, in particular and preferably, monoclonal antibodies, immunologically cross reactive to the antigens of the HTLV-I and HTLV-II. Such antibodies, for example, can be produced by injecting a mammalian or avian animal with a sufficient amount of the peptide to elicit the desired immune response and recovering said antibodies from the serum of said animals. Suitable host animals for eliciting antibodies include, for example, rabbits, horses, goats, guinea pigs, rats, mice, cows, sheep and hens. Preferably, hybridomas producing the desired monoclonal antibodies are prepared using the peptides of this invention and conventional techniques.

For example, the well-known Kohler and Milstein technique for producing monoclonal antibodies may be used. In order to distinguish monoclonal antibodies which are directed against the same antigen, but against different epitopes, the method of Stähli et al. (J. of Immunological Methods, 32, 297-304, **1980**) can be used.

Various methods which are generally known can be employed in the determination or quantification of the HTLV-I and HTLV-II or a portion thereof using the above antibodies. In one such procedure, known amounts of a serum sample or other analyte to be assayed, a radiolabeled peptide or mixture of this invention and an unlabeled peptide or mixture of this invention are mixed together, a given amount of an antipeptide antibody, preferably a monoclonal antibody, is added and the mixture allowed to stand. The resulting antibody/antigen complex is then separated from the unbound reagents by procedures known in the art, i.e., by treatment with ammonium sulphate, polyethylene glycol, a second antibody either in excess or bound to an insoluble support, or dextran-coated charcoal. The concentration of the labeled peptide is then determined in either the bound or unbound phase and the HTLV-I or HTLV-II antigen content of the sample determined by comparing the level of labeled component to a standard curve in a manner known per se.

Another suitable method for using these antibodies in assays is the "Double-Antibody-Sandwich-Assay". According to this assay, the sample to be tested is treated with two different antibodies, e.g., raised by immunizing different animals, e.g., sheep and rabbits, with a peptide or mixture of this invention. One of the antibodies is labeled and the other is coated on a solid phase. The preferred solid phase is a plastic bead and the preferred label is horse-radish peroxidase.

Typically in the "Double-Antibody-Sandwich-Assay", the sample is incubated with the solid phase antibody and the labeled antibody. However, it is also possible to contact the sample first with the solid phase antibody and then, after an optional washing, to contact the sample with the labeled antibody. Preferably, however, the sample is treated together with the solid phase and the labeled antibody. After the immunological reaction(s), the mixture is washed and the label is determined according to procedures known in the art. In the case where peroxidase is used as the label, the determination may be performed using a substrate, e.g., with o-phenylene diamine or with tetramethylbenzidine. The amount of the labeled component is proportional to the amount of the antigen(s) present in the analyte or serum sample.

The methods and assays for the determination and quantification of HTLV-I and HTLV-II antigens or antibodies against those viruses, as described above, can also be conducted in suitable test kits characterized by a peptide or mixture of this invention, or antibodies against HTLV-I or HTLV-II elicited by those peptides and mixtures.

As described above, the peptides and mixtures of this invention are also useful as the active component of vaccines capable of inducing protective immunity against the HTLV-I and HTLV-II in hosts susceptible to infection with those viruses. Routes of administration, antigen doses, number and frequency of injections will vary from individual to individual and parallel those currently being used in providing immunity to other viral infections. For example, the vaccines of this invention are pharmaceutically acceptable compositions containing at least one peptide or mixture of this invention in an amount effective in the mammal, including a human, treated with that composition to raise antibodies sufficient to protect the treated mammal from a HTLV-I or HTLV-II infection for a period of time.

The vaccines are prepared in accordance with known methods. The vaccine compositions of this invention may be conveniently and conventionally combined with physiologically acceptable carrier materials, such as pharmaceutical grade saline, tetanus toxoid, and keyhole limpet hemocyanin. The vaccine compositions of this invention may also contain adjuvants or other enhancers of immune response, such as alum preparations, liposomes or immunomodulators. Furthermore, these vaccine compositions may comprise other antigens to provide immunity against other viruses (e.g., HIV-1 and HIV-2, cytomegalovirus) or pathogens in addition to HTLV-I or HTLV-II. The amount of these other antigens is again dependent on the mammal to be treated and the course of the disease. However, the antigen should be present in an amount effective to raise antibodies sufficient to protect the treated mammal from that pathogen or virus for a period of time.

General procedures for the synthesis and utilization of the peptides of this invention are provided below.

Procedure 1: Preparation of Resins Carrying the N-FMOC Protected Amino Acid Residue

The desired N-FMOC protected amino acid residue in a mixture of methylene chloride ($CH_2Cl_2$) and dimethylformamide (DMF) (4:1) was added to a suspension of p-benzyloxy alcohol resin in $CH_2Cl_2$:DMF (4:1) at 0°C. The mixture was stirred manually for a few seconds and then treated with N,N'-dicyclohexyl-carbodiimide (DCC) followed by a catalytic amount of 4-(dimethylamino) pyridine. The mixture was stirred at 0°C for an additional 30 minutes and then at room temperature overnight. The filtered resin was washed successively with $CH_2Cl_2$, DMF and isopropanol (3 washes each) and finally, with $CH_2Cl_2$. The resin was suspended in $CH_2Cl_2$, chilled in an ice bath and redistilled pyridine was added to the stirred suspension followed by benzoyl chloride. Stirring was continued at 0°C for 30 minutes and then at room temperature for 60 minutes. After filtration, the resin was washed successively with $CH_2Cl_2$, DMF and isopropanol (3 washes each) and finally with petroleum ether (twice) before being dried under high vacuum to a constant weight.

Spectrophotometric determination of substitution according to Meienhofer et al. (Int. J. Peptide protein Res., 13, 35, 1979) indicates the degree of substitution on the resin.

Procedure 2: Coupling of Subsequent Amino Acids

The resin carrying the N-FMOC protected first amino acid residue was placed in a reaction vessel of a Biosearch 9600 Peptide Synthesizer and treated as follows:
1) Washed with DMF (4 times for 20 sec. each)
2) Prewashed with a 30% solution of piperidine in DMF (3 min.)
3) Deprotected with a 30% solution of piperidine in DMF (7 min.)
4) Washed with DMF (8 times for 20 sec. each)
5) Checked for free amino groups - Kaiser Test (must be positive)
6) The peptide resin was then gently shaken for 1 or 2 hrs with 8 equivalents of the desired FMOC-protected amino acid and 1-hydroxybenzotriazole and benzotriazol-1-yloxy-tris(dimethyl-amino)-phosphonium hexafluorophosphate all dissolved in dry redistilled DMF containing 16 equivalents of 4-methylmorpholine.
7) Washed with DMF (6 times for 20 sec. each)

After step 7, an aliquot was taken for a ninhydrin test. If the test was negative, one goes back to step 1 for coupling of the next amino acid. If the test was positive or slightly positive, steps 6 and 7 should be repeated.

The above scheme may be used for coupling each of the amino acids of the peptides described in this invention. N-protection with FMOC may also be used with each of the remaining amino acids throughout the synthesis.

Radiolabeled peptides may be prepared by incorporation of a tritiated amino acid using the above coupling protocol.

After the addition of the last amino acid, the N-FMOC of the N-terminal residue is removed by going back to steps 1-7 of the above scheme. The peptide resin is washed with $CH_2Cl_2$ and dried in vacuo to give the crude protected peptide.

Procedure 3: Deprotection and Cleavage of the Peptides from the Resin

The protected peptide-resin was suspended in a 55% solution of trifluoroacetic acid (TFA) in $CH_2Cl_2$, containing 2.5% ethanedithiol and 2.5% anisole. The mixture was flushed with $N_2$ and stirred for 1.5 hours at room temperature. The mixture was filtered and the resin washed with $CH_2Cl_2$. The resin was treated again with 20% TFA in $CH_2Cl_2$ for 5 minutes at room temperature. The mixture was filtered and the resin washed with 20% TFA in $CH_2Cl_2$ and then washed with $CH_2Cl_2$. The combined filtrates were evaporated in vacuo below 35°C and the residue triturated several times with dry dimethyl ether. The solid was dissolved in 10% aqueous acetic acid and lyophilized to afford the crude product.

The peptides containing arg and cys residues are further deprotected by HF treatment at 0°C for 1 hour in the presence of anisole and dimethylsulfide. The peptides were extracted with 10% aqueous acetic acid, washed with dimethyl ether and lyophilized to afford the crude peptides.

Procedure 4: Purification of Peptides

The crude peptides were purified by preparative HPLC on a Vydac column (2.5 X 25 mm) of $C_{18}$ or $C_4$ reverse phase with a gradient of the mobile phase. The effluent was monitored at 220 nm and subsequently by analytical HPLC. Relevant fractions were pooled, evaporated and lyophilized. The identity of the synthetic peptides was verified by analytical reverse phase chromatography and by amino acid analysis.

Procedure 5: Conjugation of Peptides to Bovine Serum Albumin or Keyhole Limpet Hemocyanin

Peptides were conjugated to BSA or KLH previously derivatized with either sulfosuccinimidyl 4-(p-maleimidophenyl) butyrate (Sulfo-SMPB) or sulfosuccinimidyl 4-(N-maleimidomethyl) cyclohexane-1-carboxylate (Sulfo-SMCC).

An aqueous solution of sulfo-SMPB or sulfo-SMCC (Pierce Chemicals) was added to a solution of BSA or KLH in 0.02 M sodium phosphate buffer (pH 7.0). The mixture was shaken at room temperature for 45 minutes and the activated carrier immediately applied to a Sephadex G-25 column equilibrated with 0.1M sodium phosphate buffer (pH 6.0) at 4°C.

The fractions of the first peak absorbance (280 nm) corresponding to activated carrier were combined in a round bottom flask to which was added a solution of peptide in 0.05 M sodium phosphate buffer (pH 6.2). The mixture was thoroughly flushed with $N_2$ and incubated overnight at room temperature. The coupling efficiency was monitored using $_3$H-labeled peptide and by amino acid analysis of the conjugate.

Procedure 6: Detection of Antibodies to HTLV-I or HTLV-II by an Enzyme Linked Immunosorbent Assay (ELISA)

Each well of the microtiter plate was saturated with 100 l of a solution containing a peptide or mixture of peptides (10 $\mu$g/ml) and left overnight. The wells were emptied and washed twice with a washing buffer (Tris, 0.043M; NaCl, 0.5M; thimerosal, 0.01% w/v; Tween 20, 0.05% v/v; pH 7.4). The wells were then saturated with 0.35 ml of washing buffer for 1 hour at 37°C and washed once with the same buffer. Serum samples to be analyzed were diluted with specimen buffer (washing buffer plus casein, 0.05% w/v). The wells were rinsed with washing buffer prior to the addition of the diluted serum sample (0.1 ml). These were left to incubate for 1 hour at room temperature. The wells were then emptied, washed twice rapidly and then once for two minutes with washing buffer. The conjugate solution (peroxidase labeled affinity purified goat antibody to human IgG or to human IgM, 0.5 mg in 5 ml 50% glycerol) diluted with 1% w/v bovine serum albumin in washing buffer was added to each well (0.1 ml) and incubated for 1 hour at room temperature. The wells were then emptied and washed five times with the washing buffer. The substrate solution (3,3', 5,5'-tetramethylbenzidine, 8 mg per ml of DMSO) was diluted with 100 volumes 0.1M citrate-acetate buffer (pH 5.6) containing 0.1% v/v of 30% $H_2O_2$ and added to each well (0.1 ml per well). After 30 minutes, the contents of each well were treated with 0.1 ml 2N $H_2SO_4$ and the optical density read at 450 nm. All determinations were done in duplicate.

Using general procedures substantially as described above, the following specific peptides were prepared: BCH-152, BCH-219, BCH-221, BCH-228, BCH-234, BCH-296, BCH-298, BCH-404, BCH-407, BCH-411, BCH-412, BCH-416, BCH-418, BCH-447, BCH-456 and BCH-486. These peptides were then evaluated for their ability to detect HTLV-I and HTLV-II-specific antibodies.

Experiment 1

In Experiment 1 individual peptides BCH-152, BCH-228, a cocktail formed by the mixture of equal amounts of BCH-152 and BCH-228, and individual peptide BCH-234 were compared in ELISA assays using a panel of seropositive and seronegative serum and plasma samples. BCH-234 is a tandem peptide wherein BCH-152 (with a Pro residue at the N-terminus) is attached to BCH-228. The results represent signal to cut off ratios and are displayed in Table 1.

TABLE 1

| SERUM NO. | W.B. | EIA WITH SYNTHETIC PEPTIDE | | | |
|---|---|---|---|---|---|
| | | 152 | 228 | 152 + 228 | 234 |
| BBI-168 | NEG | 0.50 | 0.58 | 0.50 | 0.44 |
| SC1*1/250 | POS | 8.71 | 5.94 | -- | 13.5 |
| B-12 | POS | 1.60 | 2.12 | 1.80 | 3.78 |
| B-14 | POS | 0.85 | 6.37 | 2.10 | 11.5 |
| B-18 | POS | 0.50 | 1.75 | 0.95 | 3.72 |
| B-20 | POS | 0.45 | 6.33 | 1.55 | 11.7 |
| B-21 | POS | 0.40 | 2.58 | 1.00 | 9.67 |
| B-22 | POS | 0.50 | 1.58 | 0.55 | 9.89 |
| B-23 | POS | 2.75 | 3.42 | 2.70 | 9.67 |
| B-27 | POS | 2.11 | 2.79 | 2.45 | 7.17 |
| B-30 | POS | 10.4 | 2.04 | 9.85 | 10.7 |
| B-31 | POS | 1.55 | 5.08 | 2.10 | 12.8 |
| A02-1 | POS | 0.39 | 0.45 | | 0.30 |
| A02-2 | POS | >14 | 13.7 | | >14 |
| A02-3 | POS | 0.34 | 0.34 | | 0.21 |
| A02-4 | POS | 0.75 | 0.76 | | 0.40 |
| A02-5 | POS | 0.32 | 0.39 | | 0.28 |
| A02-6 | IND** | 0.38 | 0.36 | | 0.23 |
| A02-7 | POS | 0.89 | 0.79 | | 2.72 |
| A02-8 | POS | 0.52 | 0.63 | | 0.43 |
| A02-9 | POS | 0.78 | 0.66 | | 0.47 |
| A02-10 | POS | 8.71 | 4.73 | | 10.5 |

** Indeterminable

In Table 1, the samples labeled B- were obtained from Japan and those labeled BBI-168, SC1* 1/250 and A02- are from the US. As mentioned above, infected American samples have antibody titers that are lower than those measured in blood samples from Japan.

In this experiment, the cut off above which a sample was considered positive for the presence of antibodies to HTLV-I and HTLV-II was defined as being equal to the O.D. value obtained with the negative control sample (BBI-168) plus 0.10. Results shown are signal to cut off ratios. Serum samples giving a ratio equal or above 1.0 are considered as positive.

Peptide BCH-152 recognized 8 of these 21 HTLV-I/HTLV-II seropositive samples. Peptide BCH-228 detected 14 out of 21 samples. Although peptide BCH-228 could detect all samples obtained from Japan (albeit with relatively low titers), it detected only two of the ten American samples. The origin of the sera thus lead to a net difference in sensitivity using these individual peptides.

Because peptides BCH-152 and BCH-228 represent distinct epitopes of the HTLV-I virus, we mixed them in an attempt to augment the sensitivity of detection. As indicated in Table 1, the signal measured with the mixture was, in all cases, lower than the signal measured with the best of the two peptides taken singly. There is, thus, no observed additive effect when these two peptides were mixed together.

Surprisingly, a tandem of these two peptides, e.g., peptide BCH-234, dramatically augmented the sensitivity of detection of antibodies to HTLV-I/II. In all cases, the signal/cut off ratio recorded was higher with tandem peptide BCH-234 than with the individual peptides or cocktail. BCH-234 detected 15 of the 21 samples tested. Furthermore, in addition to being able to detect an extra sample on the American panel (A02-7), the signal/cutoff ratios measured on the Japanese panel (B-series) with BCH-234 were, in all cases, higher than those obtained with the individual peptides.

Experiment 2

In Experiment 2, peptides BCH-219, BCH-221, BCH-296 and BCH-298 were tested in an ELISA using a panel of seropositive and seronegative serum and plasma samples obtained from Boston Biomedica Inc. (all U.S. strains).

The results represent signal to cut off ratios and are displayed in Table 2.

TABLE 2

| SERUM NO. | W.B. | EIA WITH SYNTHETIC PEPTIDE | | | |
|---|---|---|---|---|---|
| | | 219 | 221 | 298 | 296 |
| BBI-163 | NEG | 0.20 | 0.33 | 0.50 | 0.21 |
| SC1*1/250 | POS | 0.30 | 0.39 | 12.74 | 9.50 |
| A02-1 | POS | 11.8 | 0.41 | 4.50 | 3.71 |
| A02-2 | POS | 0.55 | 0.71 | >14 | >14 |
| A02-3 | POS | 1.20 | 0.38 | 0.85 | 0.38 |
| A02-4 | POS | 0.55 | 0.61 | 2.30 | 0.62 |
| A02-5 | POS | 1.15 | 0.53 | 2.85 | 1.67 |
| A02-6 | IND | 0.40 | 0.35 | 0.60 | 0.42 |
| A02-7 | POS | 0.35 | 0.48 | 9.45 | 7.71 |
| A02-8 | POS | 4.15 | 0.52 | 1.65 | 1.00 |
| A02-9 | POS | 11.5 | 0.51 | 1.25 | 0.75 |
| A02-10 | POS | 0.55 | 0.55 | 12.6 | 8.92 |
| A02-11 | IND | 4.40 | 0.57 | 4.35 | 2.75 |
| A02-12 | POS | 1.15 | 0.44 | 2.85 | 1.83 |
| A02-13 | IND | 0.80 | 0.43 | 1.45 | 1.04 |
| A02-14 | POS | 9.75 | 0.44 | 12.1 | 8.71 |
| A02-15 | POS | 3.30 | 0.37 | 6.00 | 2.71 |
| A02-16 | POS | 1.60 | 0.41 | 3.00 | 2.04 |
| A02-17 | IND | 0.80 | 0.39 | 1.20 | 2.75 |
| A02-18 | POS | 3.20 | 3.68 | 11.1 | 1.46 |
| A02-19 | POS | 10.0 | 0.41 | 0.85 | 2.21 |
| A02-20 | POS | 0.45 | 0.34 | >14 | >14 |
| A02-21 | POS | 4.85 | 0.38 | 3.05 | 2.71 |
| A02-22 | POS | 4.60 | 0.36 | 4.00 | 2.25 |
| A02-23 | IND | 5.15 | 0.54 | 3.95 | 2.33 |
| A02-24 | POS | >14 | 0.35 | 13.6 | 3.12 |
| A02-25 | POS | 2.55 | 0.49 | 1.75 | 1.21 |

Peptide BCH-219 detected 17 out of the 26 seropositive samples tested. More importantly, peptide BCH-219 could detect the presence of HTLV antibodies in samples that were not detectable with BCH-234 (see Table 1). Peptide BCH-298 (a-TTDNSNNSIILPPFSLAPVPPP ATRRRR-b; HTLV-II **env** 281-308), which is a longer version of peptide BCH-221 and which has a serine residue at position 285 instead of the natural cysteine residue (this was done to avoid dimerization of the peptide, to increase its stability and to increase the specificity of the test), detected 23 out of 26 samples. This latter result illustrates the importance of residues **env**(281-292) which are absent on the poorly performing peptide BCH-221 (a-PFSLAPVPPPATRRRR-b HTLV-II **env**(293-308)) and which contribute largely to the increased antigenicity of peptide BCH-298. Peptide BCH-296 (a-AIVSSPSHNSLILPPFSLSPVPTLGSRSRR-b HTLV-I **env** (283-312)) detected 22 out of 26 samples but the signals were in general lower than those obtained when using BCH-298. It was later found, however, that when peptides BCH-298 or BCH-296 were used in a cocktail, they were responsible for the detection of a large number of false positives. For example, the screening of 500 samples obtained from normal blood donors lead to 38 putative seropositives when BCH-298 was part of a three-peptide cocktail. Different peptide analogs were synthesized in order to identify and eliminate the regions on BCH-298 and on BCH-296 which were responsible for these non-specific responses. Peptides

EP 0 539 405 B1

BCH-404, BCH-407, BCH-416 and BCH-418 were particularly useful to achieving this goal (see Table 3).

Experiment 3

In Experiment 3, solutions prepared with each of the following peptides: BCH-404, BCH-407, BCH-416 and BCH-418, at 10 $\mu$g/ml, were used to coat EIA plates as described above. A cocktail of peptides was also prepared by the mixing of equal volumes of three of the peptide solutions described above (BCH-219, BCH-234 and BCH-416) and used to coat another series of plates. All seropositive samples tested were obtained from Boston Biomedica Inc.

The results represent signal to cut off ratios and are displayed in Table 3. In this experiment the cut off (0.20) is the average O.D. measured on 50 samples taken from the normal blood donor population to which were added 4 standard deviations. (Cut off = $X_{NEG}$ + 4 S.D.)

TABLE 3

| SERUM No. | EIA WITH SYNTHETIC PEPTIDE | | | | |
|---|---|---|---|---|---|
| | 404 | 407 | 416 | 418 | 219 + 234 + 416 |
| A02-01 | 0.74 | 0.81 | 7.17 | 9.21 | >23.33 |
| A02-02 | >10.37 | >23.33 | >23.33 | >23.33 | >23.33 |
| A02-03 | 0.63 | 0.31 | 0.38 | 0.38 | 1.20 |
| A02-04 | 0.87 | 1.13 | 6.25 | 0.99 | 6.31 |
| A02-05 | 0.81 | 1.34 | 2.42 | 5.26 | 7.46 |
| A02-06 | 0.66 | 0.23 | 0.24 | 0.16 | 0.66 |
| A02-07 | 7.20 | 18.78 | 18.33 | 22.92 | >23.33 |
| A02-08 | 0.67 | 0.63 | 2.53 | 2.45 | 7.42 |
| A02-09 | 0.96 | 1.58 | 7.93 | 1.72 | >23.33 |
| A02-10 | 1.15 | 7.17 | 22.33 | >23.33 | >23.33 |
| A02-11 | 0.68 | 1.39 | 7.80 | 7.78 | 16.77 |
| A02-12 | 0.77 | 1.53 | 2.74 | 6.00 | 7.87 |
| A02-13 | 0.63 | 0.49 | 2.02 | 2.37 | 5.77 |
| A02-14 | 5.78 | 15.67 | 22.28 | >23.33 | >23.33 |
| A02-15 | 2.33 | 11.32 | 4.76 | 9.52 | 16.43 |
| A02-16 | 0.71 | 0.43 | 5.38 | 6.17 | 13.39 |
| A02-17 | 0.67 | 0.46 | 1.51 | 1.79 | 2.17 |
| A02-18 | 0.69 | 0.52 | 2.95 | >23.33 | 8.06 |
| A02-19 | 0.65 | 0.37 | 0.80 | 0.66 | 7.17 |
| A02-20 | 1.10 | 10.03 | >23.33 | >23.33 | >23.33 |
| A02-21 | 0.90 | 1.59 | 3.73 | 4.95 | 9.14 |
| A02-22 | 0.91 | 1.48 | 5.86 | 7.13 | 22.23 |
| A02-23 | 1.10 | 1.98 | 5.93 | 6.97 | 18.39 |
| A02-24 | 4.13 | 22.01 | 13.82 | >23.33 | >23.33 |
| A02-25 | 0.83 | 0.98 | 2.17 | 2.56 | 5.87 |

Peptides BCH-404, BCH-407 and BCH-418 were synthesized in order to find one that would keep the same levels of sensitivity as BCH-298 (a-TTDNSNNSIILPPFSLAPVPPPATRRRR-b HTLV-II **env**(281-308)) but would not lead to false-positive signals. Peptide BCH-418 is as useful as BCH-298 in detecting the seropositive samples and did not give false-positive signals when tested on 500 serum samples taken from normal blood donors. Thus BCH-418 (which is identical to BCH-298 with the exception that the last three arginine residues were absent) is as sensitive as BCH-298 in detecting HTLV-I and HTLV-II antibodies and also much more specific. A similar observation was made with synthetic peptide BCH-416, which is a shorter version of HTLV-I peptide BCH-296. As a result, we have concluded that these three amino acid residues located near the C-terminal end of the gp46 protein of both HTLV-I and HTLV-II contribute to the binding of antibody molecules not related to HTLV-I and HTLV-II infection.

We also tested a peptide cocktail including a preferred HTLV-I tandem peptide BCH-234 and peptides derived from the sequence of HTLV-II (BCH-219 and BCH-416). The results presented in Table 3 show that this cocktail can surprisingly detect all confirmed HTLV-I and HTLV-II seropositive samples tested.

15

Specimen A02-6, which was found indeterminate by Western Blot and by immunofluorescence (IFA) was found negative in this test. That sample was found weakly positive in one test out of two.

Experiment 4

In Experiment 4, solutions prepared with synthetic peptides BCH-411 and BCH-412 at 10 $\mu$g/ml, were used to coat EIA plates as described above. Samples tested were obtained from Japan.

The results represent signal to cut off ratios and are displayed in Table 4. The cut off in this experiment is the average of O.D. values obtained with three negative samples to which 4 S.D. were added.

TABLE 4

| SERUM No. | EIA WITH SYNTHETIC PEPTIDE | |
|---|---|---|
| | 411 | 412 |
| B-12 | 1.74 | 1.34 |
| B-13 | 3.55 | 1.21 |
| B-14 | 9.84 | 2.42 |
| B-15 | 6.50 | 1.97 |
| B-16 | 2.64 | 2.24 |
| B-17 | 14.43 | 2.26 |
| B-18 | 1.81 | 1.39 |
| B-19 | 2.01 | 1.95 |
| B-20 | 7.17 | 1.47 |
| B-21 | 2.70 | 1.87 |
| B-22 | 1.78 | 1.89 |
| B-23 | 3.51 | 1.05 |
| B-24 | 4.61 | 1.55 |
| B-25 | 2.74 | 2.16 |
| B-26 | 12.09 | 2.24 |
| B-27 | 2.38 | 2.32 |
| B-28 | 10.56 | 1.92 |
| B-29 | 1.90 | 2.26 |
| B-30 | 5.39 | 2.16 |
| B-31 | 34.54 | 3.55 |

Experiment 5

In Experiment 5, peptides BCH-219, BCH-447, BCH-456 and BCH-486 were tested in ELISA using a panel of seropositive and seronegative serum and plasma samples obtained from Boston Biomedica Inc.

The results (Table 5) represent the signal to cut off ratios. The cut off is the average of the absorbancy values measured with three negative serum samples to which was added 0.100.

Peptide BCH-219 detected 18 out of the 25 seropositive samples tested (Table 5). Two of the samples (No. A02-6 and A02-17) missed by BCH-219 are WB indeterminate. In this experiment, sample A02-2, which was taken from an HTLV-I infected patient was found positive. This was not always the case. In some other experiments, this sample was not detected as positive with BCH-219. Similarly, sample A02-16, which is not detected positive in this experiment, could in other tests be detected by BCH-219.

Peptide BCH-447 detected all HTLV-I and HTLV-II samples tested. Peptide BCH-456 did not detect any of the four HTLV-I samples and was also negative with one WB-indeterminate sample (A02-6). The profile or reactivity of BCH-486 is similar to BCH-456. Although the signals measured with BCH-486 are generally higher than those obtained with BCH-456, the latter is preferred because BCH-486 gave a borderline negative signal (0.94) with one HTLV-II sample (A02-4).

16

TABLE 5

| SERUM No. | W.B. | EIA WITH SYNTHETIC PEPTIDE | | | |
|---|---|---|---|---|---|
| | | 219 | 447 | 456 | 486 |
| A02-1 | HTLV-II | 14.99 | 18.75 | 9.09 | 16.67 |
| A02-2 | HTLV-I | 1.91 | 1.81 | 0.60 | 0.50 |
| A02-3 | HTLV-II | 1.62 | 4.38 | 1.91 | 3.50 |
| A02-4 | HTLV-II | 0.73 | 1.50 | 1.30 | 0.94 |
| A02-5 | HTLV-II | 1.19 | 8.25 | 8.45 | 5.00 |
| A02-6 | Ind. | 0.51 | 1.19 | 0.15 | 0.33 |
| A02-7 | HTLV-I | 0.57 | 1.31 | 0.30 | 0.50 |
| A02-8 | HTLV-II | 3.91 | 10.75 | 5.09 | 11.17 |
| A02-9 | HTLV-II | 17.65 | 16.75 | 8.12 | 15.39 |
| A02-10 | HTLV-I | 0.52 | 1.44 | 0.39 | 0.56 |
| A02-11 | HTLV-II | 4.42 | 9.63 | 4.91 | 9.28 |
| A02-12 | HTLV-II | 1.72 | 9.06 | 7.88 | 4.72 |
| A02-13 | Ind. | 1.11 | 2.56 | 5.55 | 1.72 |
| A02-14 | HTLV-II | 10.74 | 15.25 | 8.30 | 16.67 |
| A02-15 | HTLV-II | 3.65 | 6.50 | 3.52 | 6.61 |
| A02-16 | HTLV-II | 0.58 | 12.50 | 6.12 | 12.17 |
| A02-17 | Ind. | 0.86 | 2.06 | 1.36 | 1.50 |
| A02-18 | HTLV-II | 2.95 | 6.81 | 3.15 | 5.61 |
| A02-19 | HTLV-II | 12.83 | 14.38 | 9.09 | 16.67 |
| A02-20 | HTLV-I | 0.64 | 1.25 | 0.12 | 0.22 |
| A02-21 | HTLV-II | 3.68 | 11.31 | 6.18 | 10.50 |
| A02-22 | HTLV-II | 6.45 | 9.69 | 5.33 | 10.78 |
| A02-23 | HTLV-II | 6.20 | 10.44 | 5.48 | 11.33 |
| A02-24 | HTLV-II | 17.65 | 18.75 | 9.09 | 16.67 |
| A02-25 | HTLV-II | 2.62 | 6.31 | 4.94 | 6.39 |

Experiment 6

In Experiment 6, three cocktails of peptides were prepared (10 $\mu$g total peptide/ml of solution) and used to coat EIA plates as described above. The composition of each peptide cocktail and the results obtained are described in Table 6. All samples tested were bought from Boston Biomedica Inc. and the results are expressed as signal to cut off ratios as explained before.

The first cocktail containing BCH-219 did not detect samples A02-4 and A02-6; sample A02-3 was borderline negative. Although sample A02-6 is WB-indeterminate, samples A02-3 and A02-4 are confirmed HTLV-II positive and should not be missed. The third cocktail, containing BCH-447, also missed samples 4 and 6 but could recognize sample A02-3 as positive. The signals recorded with the BCH-447 cocktail were higher than those measured using the BCH-219 cocktail. Finally, the cocktail containing BCH-456 detected all samples confirmed to be HTLV-I or HTLV-II positive and, in most cases, ratios were higher than with the other two cocktails. This experiment showed that a cocktail composed of BCH-234, BCH-456 and BCH-416 gives the best sensitivity in detecting both HTLV-I and HTLV-II seropositive samples.

In this experiment, 36 samples (No. 301 to 336) taken from a normal blood donor population were also tested. All were found negative but sample 310 which gave a strong positive signal when measured on the peptide cocktail containing BCH-219. This sample was later confirmed negative by WB. Finally, another sample, E8-2109-272, was similarly found positive with the BCH-234:BCH-219:BCH-416 cocktail although it was WB negative. This sample did not give a false signal on the other two peptide cocktails thus demonstrating their higher degree of specificity.

Experiment 6 demonstrated the superiority of peptide BCH-447, and more preferably BCH-456, over BCH-219 in increasing both the sensitivity and specificity of a peptide cocktail aimed at detecting HTLV-I and HTLV-II specific antibodies.

TABLE 6

| SERUM NO. | EIA WITH SYNTHETIC PEPTIDE | | |
|---|---|---|---|
| | 234:219:416 | 234:447:416 | 234:456:416 |
| A02-1 | 6.99 | 13.95 | 12.94 |
| A02-2 | 13.95 | 13.95 | 15.00 |
| A02-3 | 0.96 | 1.35 | 1.33 |
| A02-4 | 0.68 | 0.87 | 1.44 |
| A02-5 | 1.40 | 2.24 | 8.45 |
| A02-6 | 0.30 | 0.54 | 0.60 |
| A02-7 | 6.92 | 11.07 | 12.61 |
| A02-8 | 2.75 | 5.71 | 6.06 |
| A02-9 | 13.95 | 13.95 | 12.02 |
| A02-10 | 13.95 | 13.95 | 15.00 |
| A02-11 | 3.73 | 5.51 | 6.65 |
| A02-12 | 1.79 | 2.88 | 9.15 |
| A02-13 | 1.17 | 2.08 | 2.78 |
| A02-14 | 10.71 | 13.95 | 15.00 |
| A02-15 | 3.87 | 5.75 | 7.09 |
| A02-16 | 2.80 | 6.33 | 5.77 |
| A02-17 | 1.13 | 1.69 | 1.54 |
| A02-18 | 2.03 | 2.65 | 3.70 |
| A02-19 | 5.46 | 13.95 | 13.46 |
| A02-20 | 13.95 | 13.95 | 15.00 |
| A02-21 | 2.23 | 3.70 | 5.72 |
| A02-22 | 5.56 | 7.49 | 8.75 |
| A02-23 | 4.61 | 7.90 | 9.20 |
| A02-24 | 13.95 | 13.95 | 15.00 |
| A02-25 | 1.91 | 3.19 | 3.32 |
| 301 | 0.29 | 0.39 | 0.51 |
| 302 | 0.49 | 0.56 | 0.36 |
| 303 | 0.49 | 0.48 | 0.50 |
| 304 | 0.39 | 0.41 | 0.39 |
| 305 | 0.51 | 0.38 | 0.52 |
| 306 | 0.33 | 0.53 | 0.53 |
| 307 | 0.38 | 0.56 | 0.50 |
| 308 | 0.33 | 0.7 | 0.44 |
| 309 | 0.37 | 0.62 | 0.50 |
| 310 | 4.29 | 0.73 | 0.48 |
| 311 | 0.42 | 0.46 | 0.57 |
| 312 | 0.48 | 0.42 | 0.43 |
| 313 | 0.33 | 0.48 | 0.48 |
| 314 | 0.56 | 0.59 | 0.36 |
| 315 | 0.33 | 0.76 | 0.52 |
| 316 | 0.58 | 0.60 | 0.55 |
| 317 | 0.28 | 0.54 | 0.40 |

## TABLE 6 (con't)

### EIA WITH SYNTHETIC PEPTIDE

| SERUM No. | 234:219:416 | 234:447:416 | 234:456:416 |
|---|---|---|---|
| 318 | 0.47 | 0.61 | 0.36 |
| 319 | 0.62 | 0.56 | 0.52 |
| 320 | 0.49 | 0.51 | 0.42 |
| 321 | 0.41 | 0.57 | 0.54 |
| 322 | 0.33 | 0.52 | 0.53 |
| 323 | 0.32 | 0.71 | 0.30 |
| 324 | 0.59 | 0.53 | 0.56 |
| 325 | 0.46 | 0.56 | 0.34 |
| 326 | 0.30 | 0.44 | 0.51 |
| 327 | 0.36 | 0.43 | 0.52 |
| 328 | 0.29 | 0.40 | 0.52 |
| 329 | 0.33 | 0.51 | 0.54 |
| 330 | 0.39 | 0.55 | 0.54 |
| 331 | 0.41 | 0.56 | 0.24 |
| 332 | 0.50 | 0.60 | 0.31 |
| 333 | 0.57 | 0.67 | 0.40 |
| 334 | 0.40 | 0.53 | 0.43 |
| 335 | 0.59 | 0.47 | 0.44 |
| 336 | 0.52 | 0.59 | 0.33 |
| E8-2109-272 | 2.68 | 0.77 | 0.51 |

Experiment 7

In Experiment 7, 25 serum samples obtained from patients identified by polymerase chain reaction (PCR) as being HTLV-I (HT-100 series) or HTLV-II (HT-200 series) carriers were analyzed on plates coated with BCH-234 (HTLV-I specific) or with BCH-219 or BCH-456 (HTLV-II specific). Results displayed in Table 7 are signal to cut off ratios calculated as stated above.

Table 7 illustrates the ease with which peptides BCH-234 and BCH-456 can selectively distinguish samples obtained from a patient infected with HTLV-I from one infected with HTLV-II. Samples from the five HTLV-I infected patients give a strong signal when tested with the HTLV-I specific peptide (BCH-234) and only a low signal on BCH-219. It is noteworthy that these five samples are all negative when tested on the HTLV-II specific peptide BCH-456. Furthermore, the 20 samples taken from HTLV-II infected patients are all easily detected with BCH-456 thus showing its excellent capacity in selectively discriminating samples from HTLV-II infected patients from those taken from an HTLV-I infected one.

TABLE 7

| SERUM No. | PCR ANALYSIS | EIA WITH SYNTHETIC PEPTIDE | | |
|---|---|---|---|---|
| | | BCH-234 (HTLV-I) | BCH-219 (HTLV-II) | BCH-456 (HTLV-II) |
| HT-101 | HTLV-I | 11.11 | 0.62 | 0.85 |
| HT-102 | HTLV-I | 11.11 | 1.07 | 0.97 |
| HT-103 | HTLV-I | 11.11 | 1.92 | 0.79 |
| HT-104 | HTLV-I | 11.11 | 1.12 | 0.61 |
| HT-105 | HTLV-I | 8.72 | 1.32 | 0.75 |
| HT-201 | HTLV-II | 1.03 | 5.52 | 25.00 |
| HT-202 | HTLV-II | 1.68 | 1.86 | 16.63 |
| HT-203 | HTLV-II | 0.81 | 2.25 | 10.44 |
| HT-204 | HTLV-II | 0.75 | 3.34 | 15.07 |
| HT-205 | HTLV-II | 1.07 | 9.09 | 25.00 |
| HT-206 | HTLV-II | 1.43 | 9.09 | 25.00 |
| HT-207 | HTLV-II | 1.82 | 1.94 | 14.37 |
| HT-208 | HTLV-II | 1.75 | 5.82 | 21.03 |
| HT-209 | HTLV-II | 0.91 | 4.05 | 25.00 |
| HT-210 | HTLV-II | 0.90 | 1.58 | 6.38 |
| HT-211 | HTLV-II | 0.76 | 9.09 | 25.00 |
| HT-212 | HTLV-II | 0.67 | 6.87 | 22.54 |
| HT-213 | HTLV-II | 1.17 | 2.42 | 8.53 |
| HT-214 | HTLV-II | 1.48 | 6.87 | 25.00 |
| HT-215 | HTLV-II | 1.85 | 9.09 | 25.00 |
| HT-216 | HTLV-II | 1.09 | 2.98 | 17.37 |
| HT-217 | HTLV-II | 1.15 | 1.43 | 9.57 |
| HT-218 | HTLV-II | 1.16 | 0.67 | 1.75 |
| HT-219 | HTLV-II | 0.66 | 0.61 | 3.78 |
| HT-220 | HTLV-II | 0.99 | 5.79 | 16.75 |

While we have herein before described a number of embodiments of this invention, it is apparent that our basic constructions can be altered to provide other embodiments which utilize the processes and compositions of this invention. Therefore, it will be appreciated that the scope of this invention is to be defined by the claims appended hereto rather than by the specific embodiments which have been presented herein before by way of example.

**Claims**

**Claims for the following Contracting States : AT, BE, CH, LI, DE, DK, FR, GB, IT, LU, NL, SE**

1. A peptide having the formula

   a - X1 - b

   wherein:
   X1 is a sequence of at least six amino acids taken as a block from $AA_{283}$-$AA_{309}$ of the gp46 env protein (HTLV-I) or analogues thereof, said block comprising $AA_{294}$ and $AA_{295}$ of said gp46 env protein, or analogues of said two amino acids;
   a is an amino terminus, one to eight amino acids or a substituent effective to facilitate coupling or to improve the immunogenic or antigenic activity of the peptide; and
   b is a carboxy terminus, one to eight amino acids or a substituent effective to facilitate coupling or to improve the immunogenic or antigenic activity of the peptide.

2. A peptide having the formula

   a - X1 - b

20

wherein:

X1 is a sequence of at least 10 amino acids taken as a block from $AA_{283}$-$AA_{309}$ of the gp46 env protein (HTLV-I) or analogues thereof, said block comprising $AA_{294}$ and $AA_{295}$ of said gp46 env protein, or analogues of said two amino acids;

a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide.

3. A peptide having the formula

a - X1 - b

wherein:

X1 is a sequence of at least 15 amino acids taken as a block from $AA_{283}$-$AA_{309}$ of the gp46 env protein (HTLV-I) or analogues thereof, said block comprising $AA_{294}$ and $AA_{295}$ of said gp 46 env protein, or analogues of said two amino acids;

a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide.

4. A peptide having the formula

a - X1 - b

wherein:

X1 is a sequence of at least 20 amino acids taken as a block from $AA_{283}$-$AA_{309}$ of the gp46 env protein (HTLV-I) or analogues thereof, said block comprising $AA_{294}$ and $AA_{295}$ of said gp46 env protein, or analogues of said two amino acids;

a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide.

5. A peptide having the formula

a - X1 - b

wherein:

X1 is a sequence of at least 25 amino acids taken as a block from $AA_{283}$-$AA_{309}$ of the gp46 env protein (HTLV-I) or analogues thereof, said block comprising $AA_{294}$ and $AA_{295}$ of said gp46 env protein, or analogues of said two amino acids;

a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide.

6. The peptide according to any one of claims 1 to 5, wherein X1 is -AIVSSPSHNSLILPPFSLSPVPTLGSR- (BCH-416) and analogues thereof.

7. A peptide having the formula

a - X2 - b

wherein

X2 is a sequence of at least six amino acids taken as a block from $AA_{273}$-$AA_{305}$ of the gp46 env protein (HTLV-II) or analogues thereof;

a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide.

8.  The peptide according to claim 7, wherein X2 is selected from the group consisting of:
    -TTDNSNNSIILPPFSLAPV- (BCH-404)
    -YQPRLQAITTDNSNNSIILPPFSLAPV- (BCH-407)
    -TTDNSNNSIILPPFSLAPVPPPATR- (BCH-418)
    and analogues thereof.

9.  A peptide having the formula

    a - Z1 - b

    wherein:
    Z1 is a sequence of at least six amino acids taken as a block from $AA_{236}$-$AA_{257}$, of the gag p24 protein (HTLV-I) or analogues thereof;
    a is an amino terminus, one to eight amino acids or a substituent effective to facilitate coupling or to improve the immunogenic or antigenic activity of the peptide; and
    b is a carboxy terminus, one to eight amino acids or a substituent effective to facilitate coupling or to improve the immunogenic or antigenic activity of the peptide.

10.  A peptide according to claim 9 wherein Z1 is -QQGLRREYQQLWLAAFAALPGS- (BCH-411) and analogues thereof.

11.  A peptide of the formula

    a - Z2 - b

    wherein
    Z2 is a sequence of at least six amino acids taken as a block from $AA_{242}$-$AA_{263}$, of the gag p24 protein (HTLV-II) or analogues thereof;
    a is an amino terminus, one to eight amino acids or a substituent effective to facilitate coupling or to improve the immunogenic or antigenic activity of the peptide; and
    b is a carboxy terminus, one to eight amino acids or a substituent effective to facilitate coupling or to improve the immunogenic or antigenic activity of the peptide.

12.  A peptide according to claim 11 wherein Z2 is -QQGLRREYQNLWLAAFSTLPGN- (BCH-412) and analogues thereof.

13.  A peptide having the formula

    A - B2 - b

    wherein
    B2 is a sequence of at least six amino acids taken as a block from $AA_{171}$-$AA_{207}$ of the gp46 env protein (HTLV-II) or analogues thereof;
    a is an amino terminus, one to eight amino acids or a substituent effective to facilitate coupling or to improve the immunogenic or antigenic activity of the peptide; and
    b is a carboxy terminus, one to eight amino acids or a substituent effective to facilitate coupling or to improve the immunogenic or antigenic activity of the peptide.

14.  The peptide according to claim 13 wherein B2 is -LVHDSDLEHVLTPSTSWTTKIL- (BCH-219) and analogues thereof.

15.  The peptide according to claim 13 wherein B2 is selected from the group consisting of:
    -FITSEPTQPPPTSPPLVHDSDLEHVLTPSTS- (BCH-447)

22

-TSEPTQPPPTSPPLLVHDSDLEHVL- (BCH-456)
-TSEPTQPPPTSPPLVHDSDLEHVL- (BCH 486)
and analogues thereof.

**16.** A peptide having the formula

$$a-((((J)_n-c)_o - ((U)_p)_q - d)_r)_s - b$$

wherein:

J and U are sequences of at least six amino acids taken as a block from sequences independently selected from the group consisting of $AA_{193}-AA_{210}$ or $AA_{283}-AA_{309}$ of the gp46 **env** protein (HTLV-I); $AA_{236}-AA_{257}$ of the p24 **gag** protein (HTLV-I); $AA_{120}-AA_{130}$ of the p19 **gag** protein (HTLV-I); $AA_{171}-AA_{207}$ or $AA_{273}-AA_{305}$ of the gp46 **env** protein (HTLV-II); $AA_{242}-AA_{263}$ of the p24 **gag** protein (HTLV-II) and $AA_{126}-AA_{136}$ of the p19 **gag** protein (HTLV-II); or analogues thereof;

a is an amino terminus, one to eight amino acids or a substituent effective to facilitate coupling or to improve the immunogenic or antigenic activity of the peptide;

b is a carboxy terminus, one to eight amino acids or a substituent effective to facilitate coupling or to improve the immunogenic or antigenic activity of the peptide;

c and d, if present, are independently selected from one to eight amino acids or a substituent effective to facilitate coupling or to improve the immunogenic or antigenic activity of the peptide;

n = 1-5;
o = 1-5 if c is present, otherwise o = 1;
p = 1-5;
q = 1-5;
r = 1-5 if d is present, otherwise r = 1; and
s = 1-5.

**17.** The peptide according to claim 16 wherein $((((J)_n-c)_o - ((U)_p)_q - d)_r)_s$ is
-PHSNLDHILEPSIPWKSKPYVEPTAPQVL- (BCH-234) and analogues thereof.

**18.** A mixture or combination comprising more than one peptide according to any one of claims 1 to 17.

**19.** The mixture or combination according to claim 18 comprising at least the following peptide:
a-AIVSSPSHNSLILPPFSLSPVPTLGSR-b (BCH-416)
wherein
a is an amino terminus, one to eight amino acids or a substituent effective to facilitate coupling or to improve the immunogenic or antigenic activity of the peptide; and
b is a carboxy terminus, one to eight amino acids or a substituent effective to facilitate coupling or to improve the immunogenic or antigenic activity of the peptide;
and analogues thereof.

**20.** The mixture or combination according to claim 18 comprising at least the following peptides:
a-AIVSSPSHNSLILPPFSLSPVPTLGSR-b (BCH-416)
a-PHSNLDHILEPSIPWKSKPYVEPTAPQVL-b (BCH-234)
wherein
a is an amino terminus, one to eight amino acids or a substituent effective to facilitate coupling or to improve the immunogenic or antigenic activity of the peptide; and
b is a carboxy terminus, one to eight amino acids or a substituent effective to facilitate coupling or to improve the immunogenic or antigenic activity of the peptide;
and analogues thereof.

**21.** The mixture or combination according to claim 18 comprising at least the following peptides:
a-AIVSSPSHNSLILPPFSLSPVPTLGSR-b (BCH-416)
a-PHSNLDHILEPSIPWKSKPYVEPTAPQVL-b (BCH-234)
a-TSEPTQPPPTSPPLLVHDSDLEHVL-b (BCH-456)
wherein
a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide;
and analogues thereof.

**22.** A means for detecting the presence of antibodies to HTLV-I and/or HTVL-II antigens in an analyte, the means being characterized by a peptide according to any one of the claims 1 to 17 or by a combination or mixture according to any one of claims 18 to 21.

**23.** A method for detecting the presence of antibodies to HTLV-I and/or HTLV-II antigens in an analyte comprising the step of contacting an aliquot of the analyte with a peptide according to any one of claims 1 to 17 or a combination or mixture according to any one of claims 18 to 21.

**24.** The method according to claim 23 selected from a group consisting of ELISA, hemagglutination, single-dot or multi-dot strip assay methods.

**25.** An antibody immunologically cross reactive with a peptide or peptide mixture or combination according to any one of claims 1 to 21 or a mixture of said antibodies.

**26.** The antibody according to claim 25, the antibody being a monoclonal antibody or a mixture of said monoclonal antibodies.

**27.** A means for detecting the presence of HTLV-I and/or HTLV-II antigens in an analyte, the means being characterized by an antibody according to claims 25 or 26.

**28.** A method for detecting the presence of HTLV-I and/or HTLV-II antigens in an analyte comprising the step of contacting an aliquot of the analyte with an antibody according to claim 25 or 26.

**29.** A pharmaceutically acceptable composition comprising a peptide according to any one of claims 1 to 17 or a combination or mixture according to any one of claims 18 to 21, said peptide, combination or mixture being present in the composition in an amount effective in a mammal, including a human, treated with that composition, to raise antibodies sufficient to protect the treated mammal from HTLV-I or HTLV-II viral infections for a period of time.

**30.** The composition according to claim 29 including a physiologically acceptable carrier.

**31.** The composition according to claim 29 including an adjuvant or enhancer of the immune response.

**32.** The composition according to claim 29 including a second antigen to a pathogen other than HTLV-I or HTLV-II virus, said second antigen being present in an amount effective to raise antibodies sufficient to protect the treated mammal from infection by that pathogen for a period of time.

**33.** A composition according to any one of claims 29 to 32, for use in a method of protecting a mammal, including a human, comprising the step of treating the mammal with said composition.

**Claims for the following Contracting States : ES, GR**

**1.** A process for producing a peptide having the formula

a - X1 - b

wherein:
X1 is a sequence of at least six amino acids taken as a block from $AA_{283}$-$AA_{309}$ of the gp46 env protein (HTLV-I) or analogues thereof, said block comprising $AA_{294}$ and $AA_{295}$ of said gp46 env protein, or analogues of said two amino acids;
a is an amino terminus, one to eight amino acids or a substituent effective to facilitate coupling or to improve the immunogenic or antigenic activity of the peptide; and
b is a carboxy terminus, one to eight amino acids or a substituent effective to facilitate coupling or to improve the immunogenic or antigenic activity of the peptide, said process comprising solid state synthesis or recombinant DNA technology.

2. A process for producing a peptide having the formula

a - X1 - b

wherein:

X1 is a sequence of at least 10 amino acids taken as a block from $AA_{283}$-$AA_{309}$ of the gp46 env protein (HTLV-I) or analogues thereof, said block comprising $AA_{294}$ and $AA_{295}$ of said gp46 env protein, or analogues of said two amino acids;

a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide, said process comprising solid state synthesis or recombinant DNA technology.

3. A process for producing a peptide having the formula

a - X1 - b

wherein:

X1 is a sequence of at least 15 amino acids taken as a block from $AA_{283}$-$AA_{309}$ of the gp46 env protein (HTLV-I) or analogues thereof, said block comprising $AA_{294}$ and $AA_{295}$ of said gp46 env protein, or analogues of said two amino acids;

a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide, said process comprising solid state synthesis or recombinant DNA technology.

4. A process for producing a peptide having the formula

a - X1 - b

wherein:

X1 is a sequence of at least 20 amino acids taken as a block from $AA_{283}$-$AA_{309}$ of the gp46 env protein (HTLV-I) or analogues thereof, said block comprising $AA_{294}$ and $AA_{295}$ of said gp46 env protein, or analogues of said two amino acids;

a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide, said process comprising solid state synthesis or recombinant DNA technology.

5. A process for producing a peptide having the formula

a - X1 - b

wherein:

X1 is a sequence of at least 25 amino acids taken as a block from $AA_{283}$-$AA_{309}$ of the gp46 env protein (HTLV-I) or analogues thereof, said block comprising $AA_{294}$ and $AA_{295}$ of said gp46 env protein, or analogues of said two amino acids;

a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide, said process comprising solid state synthesis or recombinant DNA technology.

**6.** The process according to any one of claims 1 to 5, wherein X1 is -AIVSSPSHNSLILPPFSLSPVPTLGSR- (BCH-416) and analogues thereof.

**7.** A process for producing a peptide having the formula

a - X2 - b

wherein

X2 is a sequence of at least six amino acids taken as a block from $AA_{273}$-$AA_{305}$ of the gp46 env protein (HTLV-II) or analogues thereof;

a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide, said process comprising solid state synthesis or recombinant DNA technology.

**8.** The process according to claim 7, wherein X2 is selected from the group consisting of:
-TTDNSNNSIILPPFSLAPV- (BCH-404)
-YQPRLQAITTDNSNNSIILPPFSLAPV- (BCH-407)
-TTDNSNNSIILPPFSLAPVPPPATR- (BCH-418)
and analogues thereof.

**9.** A process for producing a peptide having the formula

a - Z1 - b

wherein:

Z1 is a sequence of at least six amino acids taken as a block from $AA_{236}$-$AA_{257}$, of the gag p24 protein (HTLV-I) or analogues thereof;

a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide, said process comprising solid state synthesis or recombinant DNA technology.

**10.** A process for producing a peptide according to claim 9 wherein Z1 is -QQGLRREYQQLWLAAFAALPGS- (BCH-411) and analogues thereof.

**11.** A process for producing a peptide of the formula

a - Z2 - b

wherein

Z2 is a sequence of at least six amino acids taken as a block from $AA_{242}$-$AA_{263}$, of the gag p24 protein (HTLV-II) or analogues thereof;

a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide, said process comprising solid state synthesis or recombinant DNA technology.

**12.** A process for producing a peptide according to claim 11 wherein Z2 is -QQGLRREYQNLWLAAFSTLPGN- (BCH-412) and analogues thereof.

**13.** A process for producing a peptide having the formula

A - B2 - b

wherein

B2 is a sequence of at least six amino acids taken as a block from $AA_{171}$-$AA_{207}$ of the gp46 env protein (HTLV-II) or analogues thereof;

a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide, said process comprising solid state synthesis or recombinant DNA technology.

14. The process according to claim 13 wherein B2 is - LVHDSDLEHVLTPSTSWTTKIL- (BCH-219) and analogues thereof.

15. The process according to claim 13 wherein B2 is selected from the group consisting of:

-FITSEPTQPPPTSPPLVHDSDLEHVLTPSTS- (BCH-447)

-TSEPTQPPPTSPPLLVHDSDLEHVL- (BCH-456)

-TSEPTQPPPTSPPLVHDSDLEHVL- (BCH 486)

and analogues thereof.

16. A process for producing a peptide having the formula

$$a\text{-}((((J)_n\text{-}c)_o \text{ - } ((U)_p)_q \text{ - } d)_r)_s \text{ - } b$$

wherein:

J and U are sequences of at least six amino acids taken as a block from sequences independently selected from the group consisting of $AA_{193}$-$AA_{210}$ or $AA_{283}$-$AA_{309}$ of the gp46 **env** protein (HTLV-I); $AA_{236}$-$AA_{257}$ of the p24 **gag** protein (HTLV-I); $AA_{120}$-$AA_{130}$ of the p19 **gag** protein (HTLV-I); $AA_{171}$-$AA_{207}$ or $AA_{273}$-$AA_{305}$ of the gp46 **env** protein (HTLV-II); $AA_{242}$-$AA_{263}$ of the p24 **gag** protein (HTLV-II) and $AA_{126}$-$AA_{136}$ of the p19 **gag** protein (HTLV-II); or analogues thereof;

a is an amino terminus, one to eight amino acids or a substituent effective to facilitate coupling or to improve the immunogenic or antigenic activity of the peptide;

b is a carboxy terminus, one to eight amino acids or a substituent effective to facilitate coupling or to improve the immunogenic or antigenic activity of the peptide;

c and d, if present, are independently selected from one to eight amino acids or a substituent effective to facilitate coupling or to improve the immunogenic or antigenic activity of the peptide;

n = 1-5;

o = 1-5 if c is present, otherwise o = 1;

p = 1-5;

q = 1-5;

r = 1-5 if d is present, otherwise r = 1; and

s = 1-5,

said process comprising solid state synthesis or recombinant DNA technology.

17. The process according to claim 16 wherein

$((((J)_n\text{-}c)_o \text{ - } ((U)_p)_q \text{ - } d)_r)_s$ is -PHSNLDHILEPSIPWKSKPYVEPTAPQVL- (BCH-234) and analogues thereof.

18. A process for producing a mixture or combination comprising admixing more than one peptide produced according to any one of claims 1 to 17.

19. The process according to claim 18 wherein said mixture or combination comprises at least the following peptide:

a-AIVSSPSHNSLILPPFSLSPVPTLGSR-b (BCH-416)

wherein

a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide;

and analogues thereof.

20. The process or combination according to claim 18 wherein said mixture or combination comprises at least the following peptides:

a-AIVSSPSHNSLILPPFSLSPVPTLGSR-b (BCH-416)

a-PHSNLDHILEPSIPWKSKPYVEPTAPQVL-b (BCH-234)

wherein

a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide;

and analogues thereof.

21. The process according to claim 18 wherein said mixture or combination comprises at least the following peptides:

a-AIVSSPSHNSLILPPFSLSPVPTLGSR-b (BCH-416)

a-PHSNLDHILEPSIPWKSKPYVEPTAPQVL-b (BCH-234)

a-TSEPTQPPPTSPPLLVHDSDLEHVL-b (BCH-456)

wherein

a is an amino terminus, one to eight amino acids or a substituent effective to facilitate coupling or to improve the immunogenic or antigenic activity of the peptide; and

b is a carboxy terminus, one to eight amino acids or a substituent effective to facilitate coupling or to improve the immunogenic or antigenic activity of the peptide;

and analogues thereof.

22. A means for detecting the presence of antibodies to HTLV-I and/or HTVL-II antigens in an analyte, the means being characterized by a peptide defined in any one of the claims 1 to 17 or a combination or mixture defined in any one of claims 18 to 21.

23. A method for detecting the presence of antibodies to HTLV-I and/or HTLV-II antigens in an analyte comprising the step of contacting an aliquot of the analyte with a peptide according to any one of claims 1 to 17 or a combination or mixture defined in any one of claims 18 to 21.

24. The method according to claim 23 selected from a group consisting of ELISA, hemagglutination, single-dot or multi-dot strip assay methods.

25. A process for producing an antibody immunologically cross reactive with a peptide or peptide mixture or combination defined in any one of claims 1 to 21 or a mixture of said antibodies, which comprises (a) injecting a mammalian or avian animal with a sufficient amount of the peptide to elicit the desired immune response and recovering said antibodies from the serum of said animals or (b) culturing a hybridoma.

26. The process according to claim 25, the antibody being a monoclonal antibody or a mixture of said monoclonal antibodies.

27. A means for detecting the presence of HTLV-I and/or HTLV-II antigens in an analyte, the means being characterized by an antibody produced according to claims 25 or 26.

28. A method for detecting the presence of HTLV-I and/or HTLV-II antigens in an analyte comprising the step of contacting an aliquot of the analyte with an antibody produced according to claim 25 or 26.

29. A process for producing a pharmaceutically acceptable composition comprising admixing a peptide defined in any one of claims 1 to 17 or a combination or mixture defined in any one of claims 18 to 21 with a carrier, said peptide, combination or mixture being present in the composition in an amount effective in a mammal, including a human, treated with that composition, to raise antibodies sufficient to protect the treated mammal from HTLV-I or HTLV-II viral infections for a period of time.

30. A process according to claim 29 wherein the composition includes an adjuvant or enhancer of the immune response.

**31.** A process according to claim 29 wherein the composition includes a second antigen to a pathogen other than HTLV-I or HTLV-II virus, said second antigen being present in an amount effective to raise antibodies sufficient to protect the treated mammal from infection by that pathogen for a period of time.

**32.** A composition produced according to any one of claims 29 to 31 for use in a method of protecting a mammal, including a human, comprising the step of treating the mammal with said composition.

**Patentansprüche**
**Patentansprüche für folgende Vertragsstaaten : AT, BE, CH, LI, DE, DK, FR, GB, IT, LU, NL, SE**

**1.** Peptid der Formel

a - X1 - b

in der

X1     eine als Block aus den Aminosäuren $AS_{283}$-$AS_{309}$ entnommene Sequenz von mindestens sechs Aminosäuren des gp46 env-Proteins (HTLV-I) oder Analoga derselben darstellt, wobei dieser Block die $AS_{294}$ und $AS_{295}$ des gp46 env-Proteins oder Analoga dieser beiden Aminosäuren enthält,

a     einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b     einen Carboxyterminus, eine bis acht Aminosäuren oder einen Substituenten darstellt, der dazu befähigt ist, die Kupplung zu erleichtern oder die immunogene oder antigene Aktivität des Peptids zu steigern.

**2.** Peptid der Formel

a - X1 - b

in der

X1     eine als Block aus den Aminosäuren $AS_{283}$-$AS_{309}$ entnommene Sequenz von mindestens 10 Aminosäuren des gp46 env-Proteins (HTLV-I) oder Analoga derselben darstellt, wobei dieser Block die $AS_{294}$ und $AS_{295}$ des gp46 env-Proteins oder Analoga dieser beiden Aminosäuren enthält,

a     einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b     einen Carboxyterminus, eine bis acht Aminosäuren oder einen Substituenten darstellt, der dazu befähigt ist, die Kupplung zu erleichtern oder die immunogene oder antigene Aktivität des Peptids zu steigern.

**3.** Peptid der Formel

a - X1 - b

in der

X1     eine als Block aus den Aminosäuren $AS_{283}$-$AS_{309}$ entnommene Sequenz von mindestens 15 Aminosäuren des gp46 env-Proteins (HTLV-I) oder Analoga derselben darstellt, wobei dieser Block die $AS_{294}$ und $AS_{295}$ des gp46 env-Proteins oder Analoga dieser beiden Aminosäuren enthält,

a     einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b     einen Carboxyterminus, eine bis acht Aminosäuren oder einen Substituenten darstellt, der dazu befähigt ist, die Kupplung zu erleichtern oder die immunogene oder antigene Aktivität des Peptids zu steigern.

4. Peptid der Formel

a - X1 - b

in der

X1    eine als Block aus den Aminosäuren $AS_{283}$-$AS_{309}$ entnommene Sequenz von mindestens 20 Aminosäuren des gp46 env-Proteins (HTLV-I) oder Analoga derselben darstellt, wobei dieser Block die $AS_{294}$ und $AS_{295}$ des gp46 env-Proteins oder Analoga dieser beiden Aminosäuren enthält,

a    einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b    einen Carboxyterminus, eine bis acht Aminosäuren oder einen Substituenten darstellt, der dazu befähigt ist, die Kupplung zu erleichtern oder die immunogene oder antigene Aktivität des Peptids zu steigern.

5. Peptid der Formel

a - X1 - b

in der

X1    eine als Block aus den Aminosäuren $AS_{283}$-$AS_{309}$ entnommene Sequenz von mindestens 25 Aminosäuren des gp46 env-Proteins (HTLV-I) oder Analoga derselben darstellt, wobei dieser Block die $AS_{294}$ und $AS_{295}$ des gp46 env-Proteins oder Analoga dieser beiden Aminosäuren enthält,

a    einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b    einen Carboxyterminus, eine bis acht Aminosäuren oder einen Substituenten darstellt, der dazu befähigt ist, die Kupplung zu erleichtern oder die immunogene oder antigene Aktivität des Peptids zu steigern.

6. Peptid gemäß einem der Ansprüche 1 bis 5, in welchem X1
-AIVSSPSHNSLILPPFSLSPVPTLGSR- (BCH-416)
sowie dessen Analoga darstellt.

7. Peptid der Formel

a - X2 - b

in der

X2    eine als Block aus den Aminosäuren $AS_{273}$-$AS_{305}$ entnommene Sequenz von mindestens sechs Aminosäuren des gp46 env-Proteins (HTLV-II) oder Analoga derselben darstellt,

a    einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b    einen Carboxyterminus, eine bis acht Aminosäuren oder einen Substituenten darstellt, der dazu befähigt ist, die Kupplung zu erleichtern oder die immunogene oder antigene Aktivität des Peptids zu steigern.

8. Peptid nach Anspruch 7, wobei X2 aus der aus
-TTDNSNNSIILPPFSLAPV- (BCH-404),
-YQPRLQAITTDNSNNSIILPPFSLAPV- (BCH-407),
-TTDNSNNSIILPPFSLAPVPPPATR- (BCH-418) sowie
deren Analoga
bestehenden Gruppe ausgewählt ist.

9. Peptid der Formel

a - Z1 - b

in der

Z1 eine als Block aus den Aminosäuren $AS_{236}$-$AS_{257}$ entnommene Sequenz von mindestens sechs Aminosäuren des gag p24-Proteins (HTLV-I) oder Analoga derselben darstellt,

a einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b einen Carboxyterminus, eine bis acht Aminosäuren oder einen Substituenten darstellt, der dazu befähigt ist, die Kupplung zu erleichtern oder die immunogene oder antigene Aktivität des Peptids zu steigern.

10. Peptid nach Anspruch 9, wobei Z1
-QQGLRREYQQLWLAAFAALPGS- (BCH-411)
und dessen Analoga darstellt.

11. Peptid der Formel

a - Z2 - b

in der

Z2 eine als Block aus den Aminosäuren $AS_{242}$-$AS_{263}$ entnommene Sequenz von mindestens sechs Aminosäuren des gag p24-Proteins (HTLV-II) oder Analoga derselben darstellt,

a einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b einen Carboxyterminus, eine bis acht Aminosäuren oder einen Substituenten darstellt, der dazu befähigt ist, die Kupplung zu erleichtern oder die immunogene oder antigene Aktivität des Peptids zu steigern.

12. Peptid nach Anspruch 11, wobei Z2
-QQGLRREYQNLWLAAFSTLPGN- (BCH-412)
und dessen Analoga darstellt.

13. Peptid der Formel

a - B2 - b

in der

B2 eine als Block aus den Aminosäuren $AS_{171}$-$AS_{207}$ entnommene Sequenz von mindestens sechs Aminosäuren des gp46 env-Proteins (HTLV-II) oder Analoga derselben darstellt,

a einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b einen Carboxyterminus, eine bis acht Aminosäuren oder einen Substituenten darstellt, der dazu befähigt ist, die Kupplung zu erleichtern oder die immunogene oder antigene Aktivität des Peptids zu steigern.

14. Peptid gemäß Anspruch 13, wobei B2
-LVHDSDLEHVLTPSTSWTTKIL- (BCH-219)
und Analoga desselben darstellt.

15. Peptid nach Anspruch 13, bei dem B2 aus der aus
-FITSEPTQPPPTSPPLVHDSDLEHVLTPSTS- (BCH-447),
-TSEPTQPPPTSPPLLVHDSDLEHVL- (BCH-456),
-TSEPTQPPPTSPPLVHDSDLEHVL- (BCH-486) sowie

deren Analoga bestehenden Gruppe ausgewählt ist.

16. Peptid der Formel

a - $((((J)_n\text{-}c)_o - ((U)_p)_q\text{-}d)_r)_s$ - b

in der

J und U    als Block entnommene Sequenzen von mindestens sechs Aminosäuren darstellen, die unabhängig voneinander aus der aus den Aminosäuren $AS_{193}\text{-}AS_{210}$ oder $AS_{283}\text{-}AS_{309}$ des gp46 env-Proteins (HTLV-1), den $AS_{236}\text{-}AS_{257}$ des p24 gag-Proteins (HTLV-I), den $AS_{120}\text{-}AS_{130}$ des p19 gag-Proteins (HTLV-I), den $AS_{171}\text{-}AS_{207}$ oder $AS_{273}\text{-}AS_{305}$ des gp46 env-Proteins (HTLV-II), den $AS_{242}\text{-}AS_{263}$ des p24 gag-Proteins und den $AS_{126}\text{-}AS_{136}$ des p19 gag-Proteins (HTLV-II) oder deren Analogen bestehenden Gruppe ausgewählt sind,

a    emen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b    einen Carboxyterminus, eine bis acht Aminosäuren oder einen Substituenten darstellt, der dazu befähigt ist, die Kupplung zu erleichtern oder die immunogene oder antigene Aktivität des Peptids zu steigern,

c und d,    falls vorhanden, unabhängig voneinander unter einer bis acht Aminosäuren oder einem Substituenten ausgewählt werden, der dazu befähigt ist, die Kupplung zu erleichtern oder die immunogene oder antigene Aktivität des Peptids zu steigern, und

in der gilt:

n = 1-5,
o = 1-5, wenn c vorhanden ist, sonst o = 1,
p = 1-5,
q = 1-5,
r = 1-5, wenn d vorhanden ist, sonst r = 1 und
s = 1-5.

17. Peptid gemäß Anspruch 16, wobei $((((J)_n\text{-}c)_o - ((U)_p)_q\text{-}d)_r)_s$ -PHSNLDHILEPSIPWKSKPYVEPTAPQVL- (BCH-234) und dessen Analoga darstellt.

18. Gemisch oder Kombination, enthaltend mehr als ein Peptid gemäß einem der Ansprüche 1 bis 17.

19. Gemisch oder Kombination gemäß Anspruch 18, welche mindestens das folgende Peptid enthält: a-AIVSSPSHNSLILPPFSLSPVPTLGSR-b (BCH-416), wobei

a    einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b    einen Carboxyterminus, eine bis acht Aminosäuren oder einen Substituenten darstellt, der dazu befähigt ist, die Kupplung zu erleichtern oder die immunogene oder antigene Aktivität des Peptids zu steigern,

sowie Analoga desselben.

20. Gemisch oder Kombination gemäß Anspruch 18, welche mindestens die folgenden Peptide enthält: a-AVISSPSHNSLILPPFSLSPVPTLGSR-b (BCH-416), a-PHSNLDHILEPSIPWKSKPYVEPTAPQVL-b (BCH-234) wobei

a    einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b    einen Carboxyterminus, eine bis acht Aminosäuren oder einen Substituenten darstellt, der dazu befähigt ist, die Kupplung zu erleichtern oder die immunogene oder antigene Aktivität des Peptids zu steigern,

sowie Analoga derselben.

21. Gemisch oder Kombination gemäß Anspruch 18, welche mindestens die folgenden Peptide enthält:
a-AIVSSPSHNSLILPPFSLSPVPTLGSR-b (BCH-416),
a-PHSNLDHILEPSIPWKSKPYVEPTAPQVL-b (BCH-234),
a-TSEPTQPPPTSPPLLVHDSDLEHVL-b (BCH-456)
wobei

    a    einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

    b    einen Carboxyterminus, eine bis acht Aminosäuren oder einen Substituenten darstellt, der dazu befähigt ist, die Kupplung zu erleichtern oder die immunogene oder antigene Aktivität des Peptids zu steigern,

sowie Analoga derselben.

22. Mittel (Vorrichtung oder Hilfsmittel) zum Nachweis der Anwesenheit von gegen HTLV-I- und/oder HTLV-II-Antigene gerichteten Antikörpern, gekennzeichnet durch ein Peptid gemäß einem der Ansprüche 1 bis 17 oder durch eine Kombination oder ein Gemisch gemäß einem der Ansprüche 18 bis 21.

23. Verfahren zum Nachweis der Anwesenheit von gegen HTLV-I- und/oder HTLV-II-Antigene gerichteten Antikörpern in einem Analytikum, bei dem man ein Aliquot des Analytikums mit einem Peptid gemäß einem der Ansprüche 1 bis 17 oder einer Kombination oder einem Gemisch gemäß einem der Ansprüche 18 bis 21 in Kontakt bringt.

24. Verfahren gemäß Anspruch 23, welches aus der aus ELISA, der Hämagglutination sowie dem Einzelpunkt- oder dem Mehrpunkt-Streifentest (single-dot oder multi-dot strip assay) bestehenden Gruppe ausgewählt ist.

25. Antikörper, der mit einem Peptid, einer Peptidmischung oder einer Peptidkombination gemäß einem der Ansprüche 1 bis 21 kreuzreaktiv ist, oder ein Gemisch solcher Antikörper.

26. Antikörper gemäß Anspruch 25, wobei dieser Antikörper einen monoklonalen Antikörper bzw. ein Gemisch monoklonaler Antikörper darstellt.

27. Mittel (Vorrichtung oder Hilfsmittel) zum Nachweis der Anwesenheit von HTLV-I-und/oder HTLV-II-Antigenen in einem Analytikum, welches durch einen Antikörper gemäß einem der Ansprüche 25 oder 26 gekennzeichnet ist.

28. Verfahren zum Nachweis der Anwesenheit von HTLV-I- und/oder HILV-II-Antigenen in einem Analytikum, bei dem man ein Aliquot des Analysenreagenzes mit einem Antikörper gemäß einem der Ansprüche 25 oder 26 in Kontakt bringt.

29. Pharmazeutisch annehmbare Zubereitung, welche ein Peptid gemäß einem der Ansprüche 1 bis 17 oder eine Kombination oder ein Gemisch gemäß einem der Ansprüche 18 bis 21 enthält, wobei das Peptid, die Kombination oder das Gemisch in der Zubereitung in einer wirksamen Menge vorliegt, um in einem Säuger, einschließlich des Menschen, ausreichend Antikörper zu erzeugen, um den behandelten Säuger über eine Zeitspanne hinweg vor HTLV-I- oder HTLV-II-Virusinfektionen zu schützen.

30. Zubereitung gemäß Anspruch 29, welche einen physiologisch annehmbaren Träger einschließt.

31. Zubereitung gemäß Anspruch 29, welche ein Adjuvans oder einen Verstärker der Immunantwort einschließt.

32. Zubereitung gemäß Anspruch 29, welche ein zweites Antigen zu einem anderem Pathogen als dem HTLV-I- oder HTLV-II-Virus enthält, wobei das zweite Antigen in einer wirksamen Menge vorliegt, um ausreichend Antikörper zu erzeugen, um den behandelten Säuger über eine Zeitspanne hinweg vor Infektionen durch dieses Pathogen zu schützen.

**33.** Zubereitung gemäß einem der Ansprüche 29 bis 32 zur Verwendung bei der Prophylaxe für Säuger, einschließlich des Menschen, bei dem man den Säuger mit dieser Zubereitung behandelt.

**Patentansprüche für folgende Vertragsstaaten : ES, GR**

**1.** Verfahren zur Herstellung eines Peptids der Formel

a - X1 - b

in der

X1    eine als Block aus den Aminosäuren $AS_{283}$-$AS_{309}$ entnommene Sequenz von mindestens sechs Aminosäuren des gp46 env-Proteins (HTLV-I) oder Analoga derselben darstellt, wobei dieser Block die $AS_{294}$ und $AS_{295}$ des gp46 env-Proteins oder Analoga dieser beiden Aminosäuren enthält,

a    einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b    einen Carboxyterminus, eine bis acht Aminosäuren oder einen Substituenten darstellt, der dazu befähigt ist, die Kupplung zu erleichtern oder die immunogene oder antigene Aktivität des Peptids zu steigern, wobei das Verfahren die Festphasensynthese oder rekombinante DNA-Technologie umfaßt.

**2.** Verfahren zur Herstellung eines Peptids der Formel

a - X1 - b

in der

X1    eine als Block aus den Aminosäuren $AS_{283}$-$AS_{309}$ entnommene Sequenz von mindestens 10 Aminosäuren des gp46 env-Proteins (HTLV-1) oder Analoga derselben darstellt, wobei dieser Block die $AS_{294}$ und $AS_{295}$ des gp46 env-Proteins oder Analoga dieser beiden Aminosäuren enthält,

a    einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b    einen Carboxyterminus, eine bis acht Aminosäuren oder einen Substituenten darstellt, der dazu befähigt ist, die Kupplung zu erleichtern oder die immunogene oder antigene Aktivität des Peptids zu steigern, wobei das Verfahren die Festphasensynthese oder rekombinante DNA-Technologie umfaßt.

**3.** Verfahren zur Herstellung eines Peptids der Formel

a - X1 - b

in der

X1    eine als Block aus den Aminosäuren $AS_{283}$-$AS_{309}$ entnommene Sequenz von mindestens 15 Aminosäuren des gp46 env-Proteins (HTLV-I) oder Analoga derselben darstellt, wobei dieser Block die $AS_{294}$ und $AS_{295}$ des gp46 env-Proteins oder Analoga dieser beiden Aminosäuren enthält,

a    einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b    einen Carboxyterminus, eine bis acht Aminosäuren oder einen Substituenten darstellt, der dazu befähigt ist, die Kupplung zu erleichtern oder die immunogene oder antigene Aktivität des Peptids zu steigern, wobei das Verfahren die Festphasensynthese oder rekombinante DNA-Technologie umfaßt.

**4.** Verfahren zur Herstellung eines Peptids der Formel

a - X1 - b

in der

X1     eine als Block aus den Aminosäuren $AS_{283}$-$AS_{309}$ entnommene Sequenz von mindestens 20 Aminosäuren des gp46 env-Proteins (HTLV-I) oder Analoga derselben darstellt, wobei dieser Block die $AS_{294}$ und $AS_{295}$ des gp46 env-Proteins oder Analoga dieser beiden Aminosäuren enthält,

a     einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b     einen Carboxyterminus, eine bis acht Aminosäuren oder einen Substituenten darstellt, der dazu befähigt ist, die Kupplung zu erleichtern oder die immunogene oder antigene Aktivität des Peptids zu steigern, wobei das Verfahren die Festphasensynthese oder rekombinante DNA-Technologie umfaßt.

5.    Verfahren zur Herstellung eines Peptids der Formel

a - X1 - b

in der

X1     eine als Block aus den Aminosäuren $AS_{283}$-$AS_{309}$ entnommene Sequenz von mindestens 25 Aminosäuren des gp46 env-Proteins (HTLV-I) oder Analoga derselben darstellt, wobei dieser Block die $AS_{294}$ und $AS_{295}$ des gp46 env-Proteins oder Analoga dieser beiden Aminosäuren enthält,

a     einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b     einen Carboxyterminus, eine bis acht Aminosäuren oder einen Substituenten darstellt, der dazu befähigt ist, die Kupplung zu erleichtern oder die immunogene oder antigene Aktivität des Peptids zu steigern, wobei das Verfahren die Festphasensynthese oder rekombinante DNA-Technologie umfaßt.

6.    Verfahren gemäß einem der Ansprüche 1 bis 5, in welchem X1
-AIVSSPSHNSLILPPFSLSPVPTLGSR- (BCH-416)
sowie dessen Analoga darstellt.

7.    Verfahren zur Herstellung eines Peptids der Formel

a - X2 - b

in der

X2     eine als Block aus den Aminosäuren $AS_{273}$-$AS_{305}$ entnommene Sequenz von mindestens sechs Aminosäuren des gp46 env-Proteins (HTLV-II) oder Analoga derselben darstellt,

a     einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b     einen Carboxyterminus, eine bis acht Aminosäuren oder einen Substituenten darstellt, der dazu befähigt ist, die Kupplung zu erleichtern oder die immunogene oder antigene Aktivität des Peptids zu steigern, wobei das Verfahren die Festphasensynthese oder rekombinante DNA-Technologie umfaßt.

8.    Verfahren nach Anspruch 7 (8), wobei X2 aus der aus
-TTDNSNNSIILPPFSLAPV- (BCH-404),
-YQPRLQAITTDNSNNSIILPPFSLAPV- (BCH-407),
-TTDNSNNSIILPPFSLAPVPPPATR- (BCH-418) sowie
deren Analoga
bestehenden Gruppe ausgewählt ist.

9. Verfahren zur Herstellung eines Peptids der Formel

a - Z1 - b

in der

Z1     eine als Block aus den Aminosäuren $AS_{236}$-$AS_{257}$ entnommene Sequenz von mindestens sechs Aminosäuren des gag p24-Proteins (HTLV-1) oder Analoga derselben darstellt,

a     einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b     einen Carboxyterminus, eine bis acht Aminosäuren oder einen Substituenten darstellt, der dazu befähigt ist, die Kupplung zu erleichtern oder die immunogene oder antigene Aktivität des Peptids zu steigern, wobei das Verfahren die Festphasensynthese oder rekombinante DNA-Technologie umfaßt.

10. Verfahren zur Herstellung eines Peptids nach Anspruch 9, wobei Z1
-QQGLRREYQQLWLAAFAALPGS- (BCH-411)
und dessen Analoga darstellt.

11. Verfahren zur Herstellung eines Peptids der Formel

a - Z2 - b

in der

Z2     eine als Block aus den Aminosäuren $AS_{242}$-$AS_{263}$ entnommene Sequenz von mindestens sechs Aminosäuren des gag p24-Proteins (HTLV-II) oder Analoga derselben darstellt,

a     einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b     einen Carboxyterminus, eine bis acht Aminosäuren oder einen Substituenten darstellt, der dazu befähigt ist, die Kupplung zu erleichtern oder die immunogene oder antigene Aktivität des Peptids zu steigern, wobei das Verfahren die Festphasensynthese oder rekombinante DNA-Technologie umfaßt.

12. Verfahren zur Herstellung eines Peptids nach Anspruch 11 (7), wobei Z2
-QQGLRREYQNLWLAAFSTLPGN- (BCH-412)
und dessen Analoga darstellt.

13. Verfahren zur Herstellung eines Peptids der Formel

a - B2 - b

in der

B2     eine als Block aus den Aminosäuren $AS_{171}$-$AS_{207}$ entnommene Sequenz von mindestens sechs Aminosäuren des gp46 env-Proteins (HTLV-II) oder Analoga derselben darstellt,

a     einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b     einen Carboxyterminus, eine bis acht Aminosäuren oder einen Substituenten darstellt, der dazu befähigt ist, die Kupplung zu erleichtern oder die immunogene oder antigene Aktivität des Peptids zu steigern, wobei das Verfahren die Festphasensynthese oder rekombinante DNA-Technologie umfaßt.

14. Verfahren gemäß Anspruch 13, wobei B2
-LVHDSDLEHVLTPSTSWTTKIL- (BCH-219)
und Analoga desselben darstellt.

15. Verfahren nach Anspruch 13, bei dem B2 aus der aus
-FITSEPTQPPPTSPPLVHDSDLEHVLTPSTS- (BCH-447),
-TSEPTQPPPTSPPLLVHDSDLEHVL- (BCH-456),
-TSEPTQPPPTSPPLVHDSDLEHVL- (BCH-486) sowie
deren Analoga bestehenden Gruppe ausgewählt ist.

16. Verfahren zur Herstellung eines Peptids der Formel

$$a - ((((J)_n\text{-}c)_o - ((U)_p)_q\text{-}d)_r)_s - b$$

in der

J und U   als Block entnommene Sequenzen von mindestens sechs Aminosäuren darstellen, die unabhängig voneinander aus der aus den Aminosäuren $AS_{193(6)}$-$AS_{210}$ oder $AS_{283}$-$AS_{309}$ des gp46 env-Proteins (HTLV-I), den $AS_{236}$-$AS_{257}$ des p24 gag-Proteins (HTLV-1), den $AS_{120}$-$AS_{130}$ des p19 gag-Proteins (HTLV-I), den $AS_{171}$-$AS_{207}$ oder $AS_{273}$-$AS_{305}$ des gp46 env-Proteins (HTLV-II), den $AS_{242}$-$AS_{263}$ des p24 gag-Proteins und den $AS_{126}$-$AS_{136}$ des p19 gag-Proteins (HTLV-II) oder deren Analoga bestehenden Gruppe ausgewählt sind,

a   einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b   einen Carboxyterminus, eine bis acht Aminosäuren oder einen Substituenten darstellt, der dazu befähigt ist, die Kupplung zu erleichtern oder die immunogene oder antigene Aktivität des Peptids zu steigern,

c und d,   falls vorhanden, unabhängig voneinander ausgewählt werden unter einer bis acht Aminosäuren oder einem Substituenten, der dazu befähigt ist, die Kupplung zu erleichtern oder die immunogene oder antigene Aktivität des Peptids zu steigern, und

in der gilt:

$n = 1\text{-}5$,
$o = 1\text{-}5$, wenn c vorhanden ist, sonst $o = 1$,
$p = 1\text{-}5$,
$q = 1\text{-}5$,
$r = 1\text{-}5$, wenn d vorhanden ist, sonst $r = 1$ und
$s = 1\text{-}5$,

wobei das Verfahren die Festphasensynthese oder rekombinante DNA-Technologie umfaßt.

17. Verfahren gemäß Anspruch 16 (12), wobei $((((J)_n\text{-}c)_o - ((U)_p)_q\text{-}d)_r)_s$
-PHSNLDHILEPSIPWKSKPYVEPTAPQVL- (BCH-234)
und dessen Analoga darstellt.

18. Verfahren zur Herstellung eines Gemischs oder einer Kombination, welches das Vermischen von mehr als einem gemäß einem der Ansprüche 1 bis 17 hergestellten Peptid umfaßt.

19. Verfahren gemäß Anspruch 18, wobei die Mischung oder Kombination mindestens das folgende Peptid enthält:
a-AIVSSPSHNSLILPPFSLSPVPTLGSR-b (BCH-416),
wobei

a   einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

b   einen Carboxyterminus, eine bis acht Aminosäuren oder einen Substituenten darstellt, der dazu befähigt ist, die Kupplung zu erleichtern oder die immunogene oder antigene Aktivität des Peptids zu steigern,

sowie Analoga desselben.

20. Verfahren oder Kombination gemäß Anspruch 18 (14), wobei das Gemisch oder die Kombination mindestens die folgenden Peptide enthält:
a-AIVSSPSHNSLILPPFSLSPVPTLGSR-b (BCH-416),

a-PHSNLDHILEPSIPWKSKPYVEPTAPQVL-b (BCH-234)

wobei

    a     einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

    b     einen Carboxyterminus, eine bis acht Aminosäuren oder einen Substituenten darstellt, der dazu befähigt ist, die Kupplung zu erleichtern oder die immunogene oder antigene Aktivität des Peptids zu steigern,

sowie Analoga derselben.

21. Verfahren gemäß Anspruch 18 (14), wobei das Gemisch oder die Kombination mindestens die folgenden Peptide enthält:

a-AIVSSPSHNSLILPPFSLSPVPTLGSR-b (BCH-416),

a-PHSNLDHILEPSIPWKSKPYVEPTAPQVL-b (BCH-234),

a-TSEPTQPPPTSPPLLVHDSDLEHVL-b (BCH-456)

wobei

    a     einen Aminoterminus, eine bis acht Aminosäuren oder einen Substituenten darstellt, der dazu befähigt ist, die Kupplung zu erleichtern oder die immunogene oder antigene Aktivität des Peptids zu steigern, und

    b     einen Carboxyterminus, eine bis acht Aminosäuren oder einen Substituenten darstellt, der dazu befähigt ist, die Kupplung zu erleichtern oder die immunogene oder antigene Aktivität des Peptids steigert,

sowie Analoga derselben.

22. Mittel (Vorrichtung oder Hilfsmittel) zum Nachweis der Anwesenheit von gegen HTLV-I- und/oder HTLV-II-Antigene gerichteten Antikörpern, gekennzeichnet durch ein gemäß einem der Ansprüche 1 bis 13 definiertes Peptid oder durch eine gemäß einem der Ansprüche 18 bis 21 definierte Kombination oder ein Gemisch.

23. Verfahren zum Nachweis der Anwesenheit von gegen HTLV-I- und/oder HTLV-II-Antigene gerichteten Antikörpern in einem Analytikum, bei dem man ein Aliquot des Analysenreagenzes mit einem Peptid gemäß einem der der Ansprüche 1 bis 13 oder einer Kombination oder einem Gemisch gemäß einem der Ansprüche 18 bis 21 in Kontakt bringt.

24. Verfahren gemäß Anspruch 23, welches aus der aus ELISA, der Hämagglutination sowie dem Einzelpunkt- oder dem Mehrpunkt-Streifentest (single-dot oder multi-dot strip assay) bestehenden Gruppe ausgewählt ist.

25. Verfahren zur Herstellung eines Antikörpers, der mit einem Peptid, einer Peptidmischung oder einer Peptidkombination gemäß einem der Ansprüche 1 bis 21 kreuzreaktiv ist, oder eines Gemisches solcher Antikörper, welches (a) die Injektion einer zum Stimulieren der gewünschten Immunantwort ausreichenden Menge des Peptids in ein Säugetier oder einen Vogel und Wiedergewinnung der Antikörper aus dem Serum der Tiere oder (b) die Kultivierung von Hybridomen einschließt.

26. Verfahren gemäß Anspruch 25, wobei der Antikörper einen monoklonalen Antikörper bzw. ein Gemisch monoklonaler Antikörper darstellt.

27. Mittel (Vorrichtung oder Hilfsmittel) zum Nachweis der Anwesenheit von HTLV-I-und/ oder HTLV-II-Antigenen in einem Analysenreagenz, welches durch einen gemäß einem der Ansprüche 25 oder 26 hergestellten Antikörper gekennzeichnet ist.

28. Verfahren zum Nachweis der Anwesenheit von HTLV-I- und/oder HTLV-II-Antigenen in einem Analytikum, bei dem man ein Aliquot des Analysenreagenzes mit einem gemäß einem der Ansprüche 25 oder 26 hergestellten Antikörper in Kontakt bringt.

29. Verfahren zur Herstellung einer pharmazeutisch annehmbaren Zubereitung, welches das Vermischen eines gemäß einem der Ansprüche 1 bis 17 definierten Peptids oder einer Kombination oder eines Gemisch gemäß einem der Ansprüche 18 bis 21 mit einem Träger umfaßt, wobei das Peptid, die Kombination oder das Gemisch in der Zubereitung in einer wirksamen Menge vorliegt, um in einem Säuger, einschließlich dem Menschen, ausreichend Antikörper zu erzeugen, um den behandelten Säuger über eine Zeitspanne hinweg vor HTLV-I- oder HTLV-II-Virusinfektionen zu schützen.

30. Verfahren gemäß Anspruch 29, bei dem die Zubereitung ein Adjuvans oder einem Verstärker der Immunantwort enthält.

31. Verfahren gemäß Anspruch 29, bei dem die Zubereitung ein zweites Antigen zu einem anderem Pathogen als dem HTLV-I- oder HTLV-II-Virus enthält, wobei das zweite Antigen in einer wirksamen Menge vorliegt, um ausreichend Antikörper zu erzeugen, um den behandelten Säuger über eine Zeitspanne hinweg vor Infektionen durch dieses Pathogen zu schützen.

32. Zubereitung, hergestellt gemäß einem der Ansprüche 29 bis 31, zur Verwendung bei der Prophylaxe für Säuger, einschließlich des Menschen, bei dem man den Säuger mit dieser Zubereitung behandelt.

**Revendications**
**Revendications pour les Etats contractants suivants : AT, BE, CH, LI, DE, DK, FR, IT, LU, NL, SE**

1. Peptide ayant la formule

   a - X1 - b

   dans laquelle :
   - X1 est une séquence d'au moins six acides aminés pris en bloc de $AA_{283}$-$AA_{309}$ de la protéine env gp46 (HTLV-I) ou des analogues de celle-ci, ledit bloc comprenant $AA_{294}$ et $AA_{295}$ de ladite protéine env gp46, ou des analogues desdits deux acides aminés;
   - a représente une extrémité amino terminale, un à huit acides aminés, ou un substituant efficace pour faciliter le couplage, ou pour améliorer l'activité immunogène ou antigène du peptide; et
   - b représente une extrémité carboxy terminale, un à huit acides aminés, ou un substituant efficace pour faciliter le couplage, ou pour améliorer l'activité immunogène ou antigène du peptide.

2. Peptide ayant la formule

   a- X1 - b

   dans laquelle :
   - X1 est une séquence d'au moins 10 acides aminés pris en bloc de $AA_{283}$-$AA_{309}$ de la protéine env gp46 (HTLV-I) ou des analogues de celle-ci, ledit bloc comprenant $AA_{294}$ et $AA_{295}$ de ladite protéine env gp46, ou des analogues desdits deux acides aminés;
   - a représente une extrémité amino terminale, un à huit acides aminés, ou un substituant efficace pour faciliter le couplage, ou pour améliorer l'activité immunogène ou antigène du peptide; et
   - b représente une extrémité carboxy terminale, un à huit acides aminés, ou un substituant efficace pour faciliter le couplage, ou pour améliorer l'activité immunogène ou antigène du peptide.

3. Peptide ayant la formule

   a - X1 - b

   dans laquelle :
   - X1 est une séquence d'au moins 15 acides aminés pris en bloc de $AA_{283}$-$AA_{309}$ de la protéine env gp46 (HTLV-I) ou des analogues de celle-ci, ledit bloc comprenant $AA_{294}$ et $AA_{295}$ de ladite protéine env gp46, ou des analogues desdits deux acides aminés;
   - a représente une extrémité amino terminale, un à huit acides aminés, ou un substituant efficace pour faciliter le couplage, ou pour améliorer l'activité immunogène ou antigène du peptide; et
   - b représente une extrémité carboxy terminale, un à huit acides aminés, ou un substituant efficace pour faciliter le couplage, ou pour améliorer l'activité immunogène ou antigène du peptide.

**4.** Peptide ayant la formule

a - X1 - b

dans laquelle :
- X1 est une séquence d'au moins 20 acides aminés pris en bloc de $AA_{283}$-$AA_{309}$ de la protéine env gp46 (HTLV-I) ou des analogues de celle-ci, ledit bloc comprenant $AA_{294}$ et $AA_{295}$ de ladite protéine env gp46, ou des analogues desdits deux acides aminés;
- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide.

**5.** Peptide ayant la formule

a - X1 - b

dans laquelle :
- X1 est une séquence d'au moins 25 acides aminés pris en bloc de $AA_{283}$-$AA_{309}$ de la protéine env gp46 (HTLV-I) ou des analogues de celle-ci, ledit bloc comprenant $AA_{294}$ et $AA_{295}$ de ladite protéine env gp46, ou des analogues desdits deux acides aminés;
- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide.

**6.** Peptide selon l'une quelconque des revendications 1 à 5, dans lequel X1 représente
AIVSSPSHNSLILPPFSLSPVPTLGSR- (BCH-416)
et des analogues de celui-ci.

**7.** Peptide ayant la formule

a - X2 - b

dans laquelle :
- X2 est une séquence d'au moins six acides aminés pris en bloc de $AA_{273}$-$AA_{305}$ de la protéine env gp46 (HTLV-II) ou des analogues de celle-ci;
- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide.

**8.** Peptide selon la revendication 7, dans lequel X2 est choisi au sein du groupe comprenant :
-TTDNSNNSIILPPFSLAPV- (BCH-404)
-YQPRLQAITTDNSNNSIILPPFSLAPV- (BCH-407)
-TTDNSNNSIILPPFSLAPVPPPATR- (BCH-418)
et des analogues de ceux-ci.

**9.** Peptide ayant la formule

a - Z1 - b
dans laquelle :
- Z1 est une séquence d'au moins six acides aminés pris en bloc de $AA_{236}$-$AA_{257}$ de la protéine gag p24 (HTLV-I) ou des analogues de celle-ci;
- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide.

EP 0 539 405 B1

10. Peptide selon la revendication 9, dans lequel Z1 représente
-QQGLRREYQQLWLAAFAALPGS- (BCH-411)
et des analogues de celui-ci.

11. Peptide ayant la formule

a - Z2 - b

dans laquelle :
- Z2 est une séquence d'au moins six acides aminés pris en bloc de $AA_{242}$-$AA_{263}$ de la protéine gag p24 (HTLV-II) ou des analogues de celle-ci;
- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide.

12. Peptide selon la revendication 11, dans lequel Z2 représente
-QQGLRREYQNLWLAAFSTLPGN- (BCH-412)
et des analogues de celui-ci.

13. Peptide ayant la formule

a - B2 - b

dans laquelle :
- B2 est une séquence d'au moins six acides aminés pris en bloc de $AA_{171}$-$AA_{207}$ de la protéine env gp46 (HTLV-II) ou des analogues de celle-ci;
- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide.

14. Peptide selon la revendication 13, dans lequel B2 représente
-LVHDSDLEHVLTPSTSWTTKIL- (BCH-219)
et des analogues de celui-ci.

15. Peptide selon la revendication 13, dans lequel B2 est choisi au sein du groupe comprenant :
-FITSEPTQPPPTSPPLVHDSDLEHVLTPSTS-(BCH-447)
-TSEPTQPPPTSPPLLVHDSDLEHVL- (BCH-456)
-TSEPTQPPPTSPPLVHDSDLEHVL- (BCH-486)
et des analogues de celui-ci.

16. Peptide ayant la formule :

a - $((((J)_n - c)_o - ((U)_p)_q - d)_r)_s$ - b

dans laquelle :
- J et U sont des séquences d'au moins six acides aminés pris en bloc dans des séquences choisies indépendamment au sein du groupe comprenant $AA_{193}$-$AA_{210}$ ou $AA_{283}$-$AA_{309}$ de la protéine env gp46 (HTLV-I); $AA_{236}$-$AA_{257}$ de la protéine gag p24 (HTLV-I); $AA_{120}$-$AA_{130}$ de la protéine gag p19 (HTLV-I); $AA_{171}$-$AA_{207}$ ou $AA_{273}$-$AA_{305}$ de la protéine env gp46 (HTLV-II); $AA_{242}$-$AA_{263}$ de la protéine gag p24 (HTLV-II); et $AA_{126}$-$AA_{136}$ de la protéine gag p19 (HTLV-II); ou des analogues de ceux-ci;
- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide;

41

- c et d, s'ils sont présents, sont choisis indépendamment parmi un à huit acides aminés, ou un substituant efficace pour faciliter le couplage, ou pour améliorer l'activité immunogène ou antigène du peptide;
- n = 1, 2, 3, 4, ou 5;
- o = 1, 2, 3, 4, ou 5 si c est présent, sinon o = 1;
- p = 1, 2, 3, 4, ou 5;
- q = 1, 2, 3, 4, ou 5;
- r = 1, 2, 3, 4, ou 5 si d est présent, sinon r = 1; et
- s = 1, 2, 3, 4, ou 5.

17. Peptide selon la revendication 16, dans lequel

$((((J)_n - c)_o - ((U)_p)_q - d)_r)_s$ est

-PHSNLDHILEPSIPWKSKPYVEPTAPQVL- (BCH-234)

et des analogues de celui-ci.

18. Mélange ou combinaison comprenant plus d'un peptide selon l'une quelconque des revendications 1 à 17.

19. Mélange ou combinaison selon la revendication 18, comprenant au moins le peptide suivant :

a-AIVSSPSHNSLILPPFSLSPVPTLGSR-b (BCH-416)

dans lequel :
- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide;

et les analogues de celui-ci.

20. Mélange ou combinaison selon la revendication 18, comprenant au moins les peptides suivants :

a-AIVSSPSHNSLILPPFSLSPVPTLGSR-b (BCH-416)

a-PHSNLDHILEPSIPWKSKPYVEPTAPQVL-b (BCH-234)

dans lesquels :
- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide; et les analogues de ceux-ci.

21. Mélange ou combinaison selon la revendication 18, comprenant au moins les peptides suivants :

a-AIVSSPSHNSLILPPFSLSPVPTLGSR-b (BCH-416)

a-PHSNLDHILEPSIPWKSKPYVEPTAPQVL-b (BCH-234)

a-TSEPTQPPPTSPPLLVHDSDLEHVL-b (BCH-456)

dans lesquels :
- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide;

et les analogues de ceux-ci.

22. Moyen pour détecter la présence d'anticorps des antigènes de HTLV-I et/ou HTVL-II dans un analyte, le moyen étant caractérisé par un peptide selon l'une quelconque des revendications 1 à 17, ou par une combinaison ou un mélange selon l'une quelconque des revendications 18 à 21.

23. Moyen pour détecter la présence d'anticorps des antigènes de HTLV-I et/ou HTVL-II dans un analyte, comprenant une étape de mise en contact d'une partie aliquote de l'analyte, avec un peptide selon l'une quelconque des revendications 1 à 17, ou avec une combinaison ou un mélange selon l'une quelconque des revendications 18 à 21.

**24.** Méthode selon la revendication 23, choisie au sein d'un groupe comprenant les méthodes ELISA, d'hémoagglutination, de l'essai sur bande simple point ou multipoint.

**25.** Anticorps immunologiquement réactif en mode croisé, avec un peptide ou un mélange ou une combinaison de peptides selon l'une quelconque des revendications 1 à 21, ou mélange desdits anticorps.

**26.** Anticorps selon la revendication 25, lequel anticorps est un anticorps monoclonal, ou un mélange desdits anticorps monoclonaux.

**27.** Moyen pour détecter la présence d'anticorps des antigènes de HTLV-I et/ou HTVL-II dans un analyte, le moyen étant caractérisé par un anticorps selon la revendication 25 ou la revendication 26.

**28.** Moyen pour détecter la présence d'anticorps des antigènes de HTLV-I et/ou HTVL-II dans un analyte, comprenant une étape de mise en contact d'une partie aliquote de l'analyte, avec un anticorps selon la revendication 25 ou la revendication 26.

**29.** Composition pharmaceutiquement acceptable, comprenant un peptide selon l'une quelconque des revendications 1 à 17, ou une combinaison, ou un mélange selon l'une quelconque des revendications 18 à 21, ledit peptide, ladite combinaison ou ledit mélange étant présent dans la composition en quantité efficace suffisante pour produire chez les mammifères, y compris l'homme, des anticorps pour protéger pendant une certaine période de temps, ledit mammifère traité, contre les infections virales HTLV-I ou HTLV-II.

**30.** Composition selon la revendication 29, qui comprend un véhicule physiologiquement acceptable.

**31.** Composition selon la revendication 29, qui comprend un adjuvant ou un stimulateur de la réponse immunitaire.

**32.** Composition selon la revendication 29, comprenant un second antigène d'un pathogène autre que le virus HTLV-I ou HTLV-II, ledit second antigène étant présent en quantité efficace suffisante pour produire les anticorps pour protéger pendant une certaine période de temps, l'animal traité contre l'infection due à ce pathogène.

**33.** Composition selon l'une quelconque des revendications 29 à 32, pour l'utilisation dans une méthode de protection des mammifères, y compris l'homme, comprenant l'étape de traitement du mammifère avec ladite composition.

**Revendications pour les Etats contractants suivants : ES, GR**

**1.** Procédé pour la production d'un peptide ayant la formule

a - X1 - b

dans laquelle :
- X1 est une séquence d'au moins six acides aminés pris en bloc de $AA_{283}$-$AA_{309}$ de la protéine env gp46 (HTLV-1) ou des analogues de celle-ci, ledit bloc comprenant $AA_{294}$ et $AA_{295}$ de ladite protéine env gp46, ou des analogues desdits deux acides aminés;
- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide,
ledit procédé comprenant la synthèse à l'état solide ou la technologie d'ADN recombinant.

**2.** Procédé pour la production d'un peptide ayant la formule

a - X1 - b

dans laquelle :

- X1 est une séquence d'au moins 10 acides aminés pris en bloc de $AA_{283}$-$AA_{309}$ de la protéine env gp46 (HTLV-I) ou des analogues de celle-ci, ledit bloc comprenant $AA_{294}$ et $AA_{295}$ de ladite protéine env gp46, ou des analogues desdits deux acides aminés;
- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide,

ledit procédé comprenant la synthèse à l'état solide ou la technologie d'ADN recombinant.

3. Procédé pour la production d'un peptide ayant la formule

a - X1 - b

dans laquelle :

- X1 est une séquence d'au moins 15 acides aminés pris en bloc de $AA_{283}$-$AA_{309}$ de la protéine env gp46 (HTLV-I) ou des analogues de celle-ci, ledit bloc comprenant $AA_{294}$ et $AA_{295}$ de ladite protéine env gp46, ou des analogues desdits deux acides aminés;
- a représente une extrémité amino terminale, un à huit acides aminés, ou un substituant efficace pour faciliter le couplage, ou pour améliorer l'activité immunogène ou antigène du peptide; et
- b représente une extrémité carboxy terminale un à huit acides aminés, ou un substituant efficace pour faciliter le couplage, ou pour améliorer l'activité immunogène ou antigène du peptide,

ledit procédé comprenant la synthèse à l'état solide ou la technologie d'ADN recombinant.

4. Procédé pour la production d'un peptide ayant la formule

a - X1 - b

dans laquelle :

- X1 est une séquence d'au moins 20 acides aminés pris en bloc de $AA_{283}$-$AA_{309}$ de la protéine env gp46 (HTLV-I) ou des analogues de celle-ci, ledit bloc comprenant $AA_{294}$ et $AA_{295}$ de ladite protéine env gp46, ou des analogues desdits deux acides aminés;
- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide,

ledit procédé comprenant la synthèse à l'état solide ou la technologie d'ADN recombinant.

5. Procédé pour la production d'un peptide ayant la formule

a - X1 - b

dans laquelle :

- X1 est une séquence d'au moins 25 acides aminés pris en bloc de $AA_{283}$-$AA_{309}$ de la protéine env gp46 (HTLV-I) ou des analogues de celle-ci, ledit bloc comprenant $AA_{294}$ et $AA_{295}$ de ladite protéine env gp46, ou des analogues desdits deux acides aminés;
- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide,

ledit procédé comprenant la synthèse à l'état solide ou la technologie d'ADN recombinant.

6. Procédé selon l'une quelconque des revendications 1 à 5, dans lequel X1 représente
AIVSSPSHNSLILPPFSLSPVPTLGSR- (BCH-416)
et des analogues de celui-ci.

7. Procédé pour la production d'un peptide ayant la formule

a - X2 - b

dans laquelle :
- X2 est une séquence d'au moins six acides aminés pris en bloc de $AA_{273}$-$AA_{305}$ de la protéine env gp46 (HTLV-II) ou des analogues de celle-ci;
- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide,

ledit procédé comprenant la synthèse à l'état solide ou la technologie d'ADN recombinant.

8. Peptide selon la revendication 7, dans lequel X2 est choisi au sein du groupe comprenant :
-TTDNSNNSIILPPFSLAPV- (BCH-404)
-YQPRLQAITTDNSNNSIILPPFSLAPV- (BCH-407)
-TTDNSNNSIILPPFSLAPVPPPATR- (BCH-418)
et des analogues de ceux-ci.

9. Procédé pour la production d'un peptide ayant la formule

a - Z1 - b

dans laquelle :
- Z1 est une séquence d'au moins six acides aminés pris en bloc de $AA_{236}$-$AA_{257}$ de la protéine gag p24 (HTLV-I) ou des analogues de celle-ci;
- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide,

ledit procédé comprenant la synthèse à l'état solide ou la technologie d'ADN recombinant.

10. Procédé pour la production d'un peptide selon la revendication 9, dans lequel Z1 représente
-QQGLRREYQQLWLAAFAALPGS- (BCH-411)
et des analogues de celui-ci.

11. Procédé pour la production d'un peptide de formule :

a - Z2 - b

dans laquelle :
- Z2 est une séquence d'au moins six acides aminés pris en bloc de $AA_{242}$-$AA_{263}$ de la protéine gag p24 (HTLV-II) ou des analogues de celle-ci;
- a représente une extrémité amino terminale, un à huit acides amitiés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide,

ledit procédé comprenant la synthèse à l'état solide ou la technologie d'ADN recombinant.

12. Procédé pour la production d'un peptide selon la revendication 11 dans lequel Z2 représente
-QQGLRREYQNLWLAAFSTLPGN- (BCH-412)
et des analogues de celui-ci.

13. Procédé pour la production d'un peptide ayant la formule

a - B2 - b

dans laquelle :
- B2 est une séquence d'au moins six acides aminés pris en bloc de $AA_{171}$-$AA_{207}$ de la protéine env gp46 (HTLV-II) ou des analogues de celle-ci;

- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide,

ledit procédé comprenant la synthèse à l'état solide ou la technologie d'ADN recombinant.

14. Procédé pour la production d'un peptide selon la revendication 13, dans lequel B2 représente
    -LVHDSDLEHVLTPSTSWTTKIL- (BCH-219)
    et des analogues de celui-ci.

15. Procédé selon la revendication 13, dans lequel B2 est choisi au sein du groupe comprenant :
    -FITSEPTQPPPTSPPLVHDSDLEHVLTPSTS-(BCH-447)
    -TSEPTQPPPTSPPLLVHDSDLEHVL- (BCH-456)
    -TSEPTQPPPTSPPLVHDSDLEHVL- (BCH-486)
    et des analogues de celui-ci.

16. Procédé pour la production d'un peptide ayant la formule :

    $a - ((((J)_n - c)_o - ((U)_p)_q - d)_r)_s - b$

    dans laquelle :
    - J et U sont des séquences d'au moins six acides aminés pris en bloc dans des séquences choisies indépendamment au sein du groupe comprenant $AA_{193}$-$AA_{210}$ ou $AA_{283}$-$AA_{309}$ de la protéine env gp46 (HTLV-I); $AA_{236}$-$AA_{257}$ de la protéine gag p24 (HTLV-I); $AA_{120}$-$AA_{130}$ de la protéine gag p19 (HTLV-1); $AA_{171}$-$AA_{207}$ ou $AA_{273}$-$AA_{305}$ de la protéine env gp46 (HTLV-II); $AA_{242}$-$AA_{263}$ de la protéine gag p24 (HTLV-II); et $AA_{126}$-$AA_{136}$ de la protéine gag p19 (HTLV-II); ou des analogues de ceux-ci;
    - a représente une extrémité amino terminale, un à huit acides aminés, ou un substituant efficace pour faciliter le couplage, ou pour améliorer l'activité immunogène ou antigène du peptide; et
    - b représente une extrémité carboxy terminale, un à huit acides aminés, ou un substituant efficace pour faciliter le couplage, ou pour améliorer l'activité immunogène ou antigène du peptide;
    - c et d, s'ils sont présents, sont choisis indépendamment parmi un à huit acides aminés, ou un substituant efficace pour faciliter le couplage, ou pour améliorer l'activité immunogène ou antigène du peptide;
    - n = 1, 2, 3, 4, ou 5;
    - o = 1, 2, 3, 4, ou 5 si c est présent, sinon o = 1;
    - p = 1, 2, 3, 4, ou 5;
    - q = 1, 2, 3, 4, ou 5;
    - r = 1, 2 3, 4, ou 5 si d est présent, sinon r = 1; et
    - s = 1, 2, 3, 4, ou 5;
    ledit procédé comprenant la synthèse à l'état solide ou la technologie d'ADN recombinant.

17. Procédé selon la revendication 16 dans lequel
    $((((J)_n - c)_o - ((U)_p)_q - d)_r)_s$ est
    -PHSNLDHILEPSIPWKSKPYVEPTAPQVL- (BCH-234)
    et des analogues de celui-ci.

18. Procédé pour la production d'un mélange ou d'une combinaison comprenant plus d'un peptide produit selon l'une quelconque des revendications 1 à 17.

19. Procédé selon la revendication 18, dans lequel ledit mélange ou ladite combinaison comprend au moins le peptide suivant :
    a-AIVSSPSHNSLILPPFSLSPVPTLGSR-b (BCH-416)
    dans lequel :
    - a représente une extrémité amino terminale, un à huit acides aminés, ou un substituant efficace pour faciliter le couplage, ou pour améliorer l'activité immunogène ou antigène du peptide; et
    - b représente une extrémité carboxy terminale, un à huit acides aminés, ou un substituant efficace pour faciliter le couplage, ou pour améliorer l'activité immunogène ou antigène du peptide;

et les analogues de celui-ci.

20. Procédé selon la revendication 18 dans lequel ledit mélange ou ladite combinaison comprend au moins les peptides suivants :

       a-AIVSSPSHNSLILPPFSLSPVPTLGSR-b (BCH-416)

       a-PHSNLDHILEPSIPWKSKPYVEPTAPQVL-b (BCH-234)

dans lesquels :

- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides amines, ou un substituant efficace pour faciliter le couplage, ou pour améliorer l'activité immunogène ou antigène du peptide;

et les analogues de ceux-ci.

21. Procédé selon la revendication 18 dans lequel ledit mélange ou ladite combinaison comprend au moins les peptides suivants :

       a-AIVSSPSHNSLILPPFSLSPVPTLGSR-b (BCH-416)

       a-PHSNLDHILEPSIPWKSKPYVEPTAPQVL-b (BCH-234)

       a-TSEPTQPPPTSPPLLVHDSDLEHVL-b (BCH-456)

dans lesquels :

- a représente une extrémité amino terminale, un à huit acides aminés, ou un substituant efficace pour faciliter le couplage, ou pour améliorer l'activité immunogène ou antigène du peptide; et
- b représente une extrémité carboxy terminale, un à huit acides aminés, ou un substituant efficace pour faciliter le couplage, ou pour améliorer l'activité immunogène ou antigène du peptide; et

et les analogues de ceux-ci.

22. Moyen pour détecter la présence d'anticorps des antigènes de HTLV-I et/ou HTVL-II dans un analyte, le moyen étant caractérisé par un peptide défini dans l'une quelconque des revendications 1 à 17 ou par une combinaison ou un mélange défini dans l'une quelconque des revendications 18 à 21.

23. Méthode pour détecter la présence d'anticorps des antigènes de HTLV-I et/ou HTVL-II dans un analyte, comprenant une étape de mise en contact d'une partie aliquote de l'analyte, avec un peptide selon l'une quelconque des revendications 1 à 17 ou avec une combinaison ou un mélange défini dans l'une quelconque des revendications 18 à 21.

24. Méthode selon la revendication 23, choisie au sein d'un groupe comprenant les méthodes ELISA, d'hémoagglutination, de l'essai sur bande simple point ou multipoint.

25. Procédé pour la production d'un anticorps immunologiquement réactif en mode croisé, avec un peptide ou un mélange ou une combinaison de peptides selon l'une quelconque des revendications 1 à 21, ou mélange desdits anticorps, qui comprend (a) l'injection à un animal, mammifère ou oiseau, d'une quantité de peptide suffisante pour provoquer la réponse immunitaire désirée, et la récupération desdits anticorps à partir du sérum desdits animaux, ou (b) la culture d'un hybridome.

26. Procédé selon la revendication 25, dans lequel l'anticorps est un anticorps monoclonal, ou un mélange desdits anticorps monoclonaux.

27. Moyen pour détecter la présence d'anticorps des antigènes de HTLV-I et/ou HTVL-II dans un analyte, le moyen étant caractérisé par un anticorps selon la revendication 25 ou la revendication 26.

28. Moyen pour détecter la présence d'anticorps des antigènes de HTLV-I et/ou HTVL-II dans un analyte, comprenant une étape de mise en contact d'une partie aliquote de l'analyte, avec un anticorps selon la revendication 25 ou la revendication 26.

29. Procédé pour la production d'une composition pharmaceutiquement acceptable, comprenant le mélange d'un peptide défini dans l'une quelconque des revendications 1 à 17, ou d'une combinaison, ou d'un mélange défini dans l'une quelconque des revendications 18 à 21, avec un véhicule, ledit peptide, ladite combinaison ou ledit mélange étant présent dans la composition en quantité efficace chez le mammifère, y compris l'homme, traité avec cette composition, pour produire assez d'anticorps pour protéger pendant une certaine période de temps, ledit mammifère traité, contre les infections virales HTLV-I ou HTLV-II.

30. Procédé selon la revendication 29, dans lequel la composition comprend un adjuvant ou un stimulateur de la réponse immunitaire.

31. Procédé selon la revendication 29, dans lequel la composition comprend un second antigène à un pathogène autre que le virus HTLV-1 ou HTLV-II, ledit second antigène étant présent en quantité efficace pour produire assez d'anticorps pour protéger pendant une certaine période de temps, l'animal traité contre l'infection due à ce pathogène.

32. Composition produite selon selon l'une quelconque des revendications 29 à 31, pour l'utilisation dans une méthode de protection des mammifères, y compris l'homme, comprenant l'étape de traitement du mammifère avec ladite composition.

# FIG. IA

## ENVELOPE PROTEINS

```
HTLV-I   1 MGKFLATLILFFQFCPLIFGDYSPSCCTLTIGVSSYHSKPCNPAQPVCSW
HTLV-II  1 MGNVF..FLLLFSLTHFPLAQ..QSRCTLTIGISSYHSSPCSPTQPVCTW

        51 TLDLLALSADQALQPPCPNLVSYSSYHATYSLYLFPHWTKKPNRNGGGYY
        47 NLDLNSLTTDQRLHPPCPNLITYSGFHKTYSLYLFPHWIKKPNRQGLGYY

       101 SASYSDPCSLKCPYLGCQSWTCPYTGAVSSPYWKFQHDVNFTQEVSRLNI
        97 SPSYNDPCSLQCPYLGCQAWTSAYTGPVSSPSWKFHSDVNFTQEVSQVSL

       151 NLHFSKCGFPFSLLVDAPGYDPIWFLNTEPSQLPPTAPPLLPHSNLDHIL
       147 RLHFSKCGSSMTLLVDAPGYDPLWFITSEPTQPPPTSPPLVHDSDLEHVL
```

EP 0 539 405 B1

# FIG. 1B

201 EPSIPWKSKLLTLVQLTLQSTNYTCIVCIDRASLSTWHVLYSPNVSVP.S
197 TPSTSWTTKILKFIQLTLQSTNYSCMVCVDRSSLSSWHVLYTPNISIPQQ

250 SSSTPLLYPSLALPAPHLTLPFNWTHCFDPQIQAIVSSPCHNSLILPPFS
247 TSSRTILFPSLALPAPP.SQPFPWTHCYQPRLQAITTDNCNNSIILPPFS

                gp46 <> gp21
300 LSPVPTLGSRSRRAVPVAVWLVSALAMGAGVAGGITGSMSLASGKSLLHE
296 LAPVPPPATRRRRAVPIAVWLVSALAAGTGIAGGVTGSLSLASSKSLLLE
                gp46 <> gp21

350 VDKDISQLTQAIVKNHKNLLKIAQYAAQNRRGLDLLFWEQGGLCKALQEQ
346 VDKDISHLTQAIVKNHQNILRVAQYAAQNRRGLDLLFWEQGGLCKAIQEQ

400 CRFPNITNSHVPILQERPPLENRVLTGWGLNWDLGLSQWAREALQTGITL
396 CCFLNISNTHVSVLQERPPLEKRVITGWGLNWDLGLSQWAREALQTGITI

450 VALLLLVILAGPCILRQLRHLPSRV..RYPHYSLIKPESSL
446 LALLLLVILFGPCILRQIQALPQRLQNRHNQYSLINPETML

EP 0 539 405 B1

# FIG. 2A

## GAG PROTEINS

```
HTLV-I   1 MGQIFSRSASPIPRPPRGLAAHHWLNFLQAAYRLEPGPSSYDFHQLKKFL
HTLV-II  1 MGQIHGLSPTPIPKAPRGLSTHHWLNFLQAAYRLQPRPSDFDFQQLRRFL


        51 KIALETPARICPINYSLLASLLPKGYPGRVNEILHILIQTQAQIPSRPA?
        51 KLALKTPIWLNPIDYSLLASLIPKGYPGRVVEIINILVKNQVSPSAPAAP

                                        p19 <> p24
       101 ......PPPSSPTHDPPDSDPQIPPPYVEPTAPQVLPVMHPHGAPPNHRP
       101 VPTPICPTTTPPPPPPPSPEAHVPPPYVEPTTTQCFPILHPPGAPSAHRP
                                        p15 <> p24


       145 WQMKDLQAIKQEVSQAAPGSPQFMQTIRLAVQQFDPTAKDLQDLLQYLCS
       151 WQMKDLQAIKQEVSSSALGSPQFMQTLRLAVQQFDPTAKDLQDLLQYLCS
```

EP 0 539 405 B1

## FIG. 2B

```
195  SLVASLHHQQLDSLISEAETRGITGYNPLAGPLRVQANNPQQQGLRREYQ
201  SLVVSLHHQQLNTLITEAETRGMTGYNPMAGPLRMQANNPAQQGLRREYQ


245  QLWLAAFAALPGSAKDPSWASILQGLEEPYHAFVERLNIALDNGLPEGTP
251  NLWLAAFSTLPGNTRDPSWAAILQGLEEPYCAFVERLNVALDNGLPEGTP


295  KDPILRSLAYSNANKECQKLLQARGHTNSPLGDMLRACQTWTPKDKTKVL
301  KEPILRSLAYSNANKECQKILQARGHTNSPLGEMLRTCQAWTPKDKTKVL

     > p15
345  VVQPKKPPPNQPCFRCGKAGHWSRDCTQPRPPPGPCPLCQDPTHWKRDCP
351  VVQPRRPPPTQPCFRCGKVGHWSRDCTQPRPPPGPCPLCQDPSHWKRDCP
     > p12

395  RLKPTIPEPEPEEDALLLDLP..ADIPHPKNSIGGEV
401  QLKP....PQEEGEPLLLDLPSTSGTTEEKNSLRGEI
```

EP 0 539 405 B1